# EUROPEAN PATENT APPLICATION

(11) **EP 4 825 189 A1**
(43) Date of publication of application: **21.10.2026**
(21) Application number: 26153594.2
(22) Date of filing: 15.05.2020
(51) Int. Cl.: A23P 10/30, A23J 3/08, A23J 3/10, A23K 20/00, A23K 40/30, A23L 33/10, A23C 9/13, A23C 9/152, A61K 9/50

(54) **METHODS FOR ENCAPSULATION**

(30) Priority: 16.05.2019 EP 19174946
(62) Document number(s) of the earlier application(s) in accordance with Art. 76 EPC: 20726115.7 / 3 968 789
(71) Applicant: Chr. Hansen A/S, 2970 Hoersholm (DK)
(72) Inventor: DHAYAL, Surender Kumar, 2970 Hoersholm (DK); LUND, Martin, 2970 Hoersholm (DK); BISGAARD-FRANTZEN, Henrik, 2970 Hoersholm (DK); VAN DEN BRINK, Johannes Maarten, 2970 Hoersholm (DK)
(74) Representative: NVS EPO Representatives

(57) **Abstract**

The present invention relates to methods for encapsulating a bioactive agent such as a microorganism, methods for making a modified food, feed, cosmetic, plant health, seed health or pharmaceutical product comprising heteropolymer particles encapsulating a bioactive agent such as a microorganism, compositions and modified products comprising heteropolymer particles of a phenolic compound and a protein, and a bioactive agent encapsulated therein obtainable by such methods. Also provided is a method for delivering a bioactive agent to a subject, comprising administering to said subject the modified food, feed, cosmetic, plant health, seed health or pharmaceutical product described herein.

## Description

### TECHNICAL FIELD

The present invention relates to methods for encapsulating a bioactive agent such as a microorganism, methods for making a modified food, feed, cosmetic, plant health, seed health or pharmaceutical product comprising heteropolymer particles encapsulating a bioactive agent such as a microorganism, compositions and modified products comprising heteropolymer particles of a phenolic compound and a protein, and a bioactive agent encapsulated therein obtainable by such methods. Also provided is a method for delivering a bioactive agent to a subject, comprising administering to said subject the modified food, feed, cosmetic, plant health, seed health or pharmaceutical product described herein.

### BACKGROUND

Encapsulation of bioactive agents, in particular for applications in the food, feed or pharmaceutical industry, has the potential to deliver bioactive agents to a subject into the gut, in particular probiotics. Because of the promising preclinical and clinical results, probiotics have been incorporated into a range of products. However, there are still many challenges to overcome with respect to the microencapsulation process as well as to survival of the probiotics during delivery.

In the context of probiotic encapsulation, techniques that are gentle and non-aggressive towards the cells are required. Encapsulation by spray-drying, freeze-drying or fluidized bed drying have shown their limitations because the cells encapsulated by these techniques are completely released into the product. Thereby, the cells are not protected towards the food matrix environment and in the presence of gastric fluid or bile.

Methods for encapsulating bioactive agents which are reliable and easy to perform are thus needed.

### SUMMARY

The invention is as defined in the claims.

Herein is provided a method for encapsulating a bioactive agent such as a microorganism, said method comprising the steps of:
i) providing a bioactive agent, a heteropolymer obtained by cross-linking of a protein comprising at least one aromatic amino acid with a phenolic compound such as a polyphenolic compound, and a polymer, said polymer having the ability to phase separate from or with said heteropolymer, preferably having the ability to coacervate or to form a complex with said heteropolymer;
ii) contacting said bioactive agent with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent,
thereby obtaining a product comprising heteropolymer particles encapsulating said bioactive agent.

Also provided is a method for making a modified food, feed, cosmetic, plant health, seed health or pharmaceutical product comprising heteropolymer particles encapsulating a bioactive agent such as a microorganism, said methods comprising the steps of:
i) providing a bioactive agent such as a microorganism, a heteropolymer obtained by cross-linking of a protein comprising at least one aromatic amino acid with a phenolic compound such as a polyphenolic compound, and a polymer such as a biopolymer, said polymer having the ability to phase separate from or with said heteropolymer, preferably having the ability to coacervate or to form a complex with said heteropolymer;
ii) contacting said bioactive agent with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent,
   wherein
   - if the protein is provided in a first substrate and the phenolic compound is provided in a second substrate, one of the first and second substrate is the food to be modified, steps ii) and iii) are performed within said first or second substrate, and the modified food, feed, cosmetic, plant health, seed health or pharmaceutical product is obtained in step iii); or
   - if the protein and the phenolic compound are provided in a composition, steps ii) and iii) are performed within said composition, a composition comprising heteropolymers encapsulating said bioactive agent is obtained in step iii), and the method further comprises a step of:
iv) adding the composition comprising heteropolymer particles encapsulating said bioactive agent to a food, feed, cosmetic, plant health, seed health or pharmaceutical product to be modified;
thereby obtaining a modified food, feed, cosmetic, plant health, seed health or pharmaceutical product comprising heteropolymer particles encapsulating said bioactive agent.

Also provided is a composition comprising heteropolymer particles of a phenolic compound and a protein, and a bioactive agent encapsulated therein, obtainable by the methods described herein.

Also provided is a modified product comprising heteropolymer particles of a phenolic compound and a protein, and a bioactive agent encapsulated therein, obtainable by the methods described herein, wherein the modified product is a modified food, feed, cosmetic, plant health, seed health or pharmaceutical product.

Also provided is a method for delivering a bioactive agent to a subject, said method comprising administering to said subject a modified food, feed, cosmetic, plant health, seed health or pharmaceutical product as described herein.

### DESCRIPTION OF THE DRAWINGS

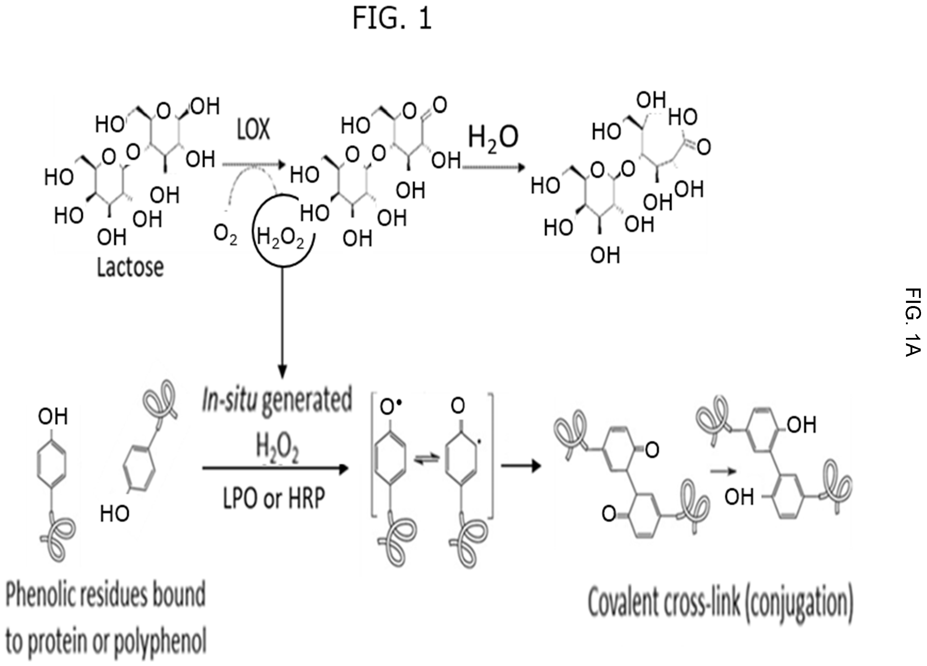

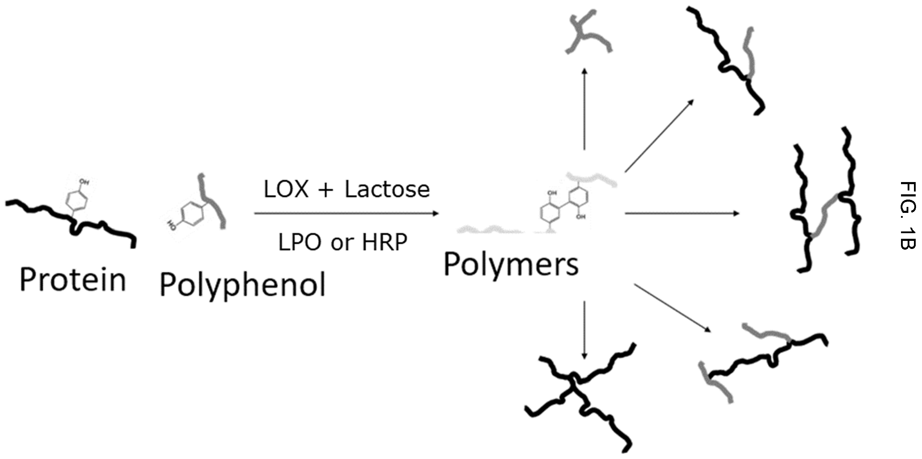

**Figure 1****:** Schematic diagram of cross-linking/polymerization/modifications of a protein that contains phenolic residues such as tyrosine and/or a polyphenolic compound, using a combination of lactose oxidase (LOX), lactose, peroxidase e.g. horseradish peroxidase (HRP) or lactoperoxidase (LPO) **(A).** Various types of covalently conjugated products (polymers: covalently cross-linked polymers) can be formed by varying the relative amounts of the protein and polyphenolic substrates **(B).**

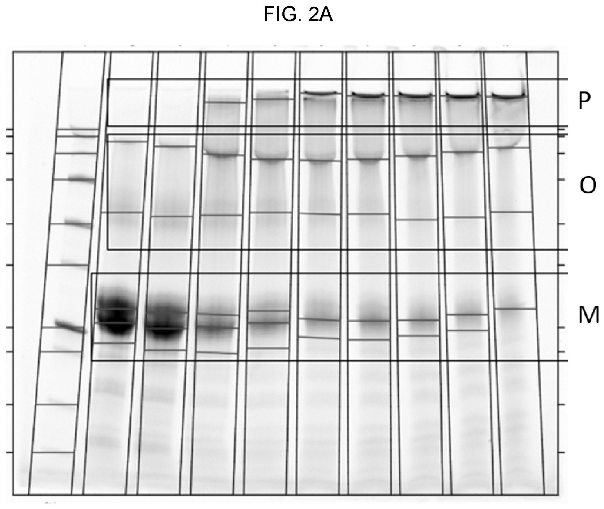

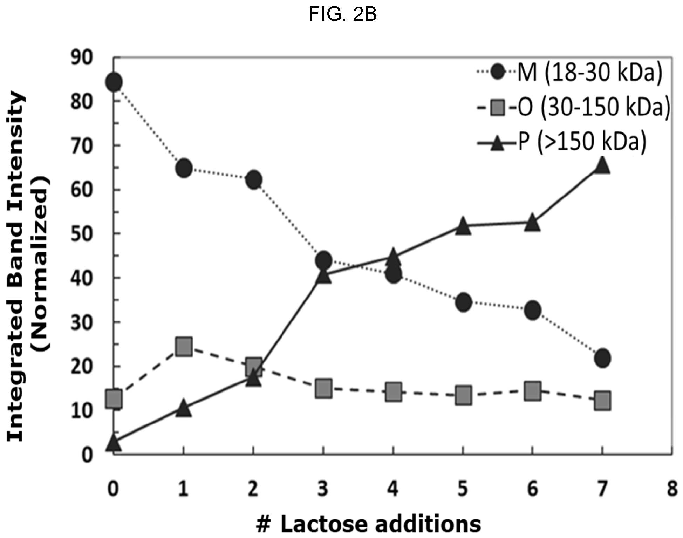

**Figure 2****:** Polymerization of sodium caseinate due to covalent cross-linking induced by using a combination of lactose oxidase (LOX) and horseradish peroxidase (HRP). The reaction was controlled by sequential dosing of lactose. No lactose was added to the blank sample, whereas the lactose was sequentially dosed in the test samples (up to 7 additions). SDS-PAGE **(A)** and integrated intensity of the bands in SDS-PAGE **(B).** P: polymers; O: oligomers; M: monomers. **(A):** lanes contained (from left to right): marker, blank, blank + enzymes, L1, L2, L3, L4, L5, L6, L7.

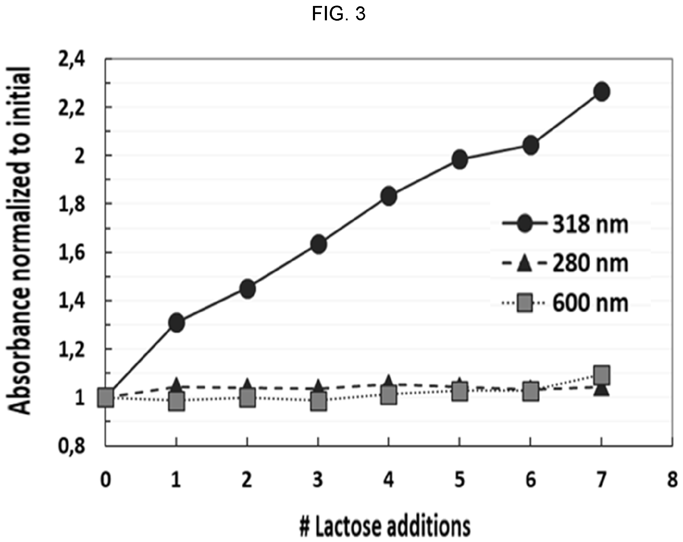

**Figure 3****:** Increase in absorbance at 318 nm indicating formation of oligo-tyrosine (e.g. di-tyrosine) cross-links for the samples described in figure 2.

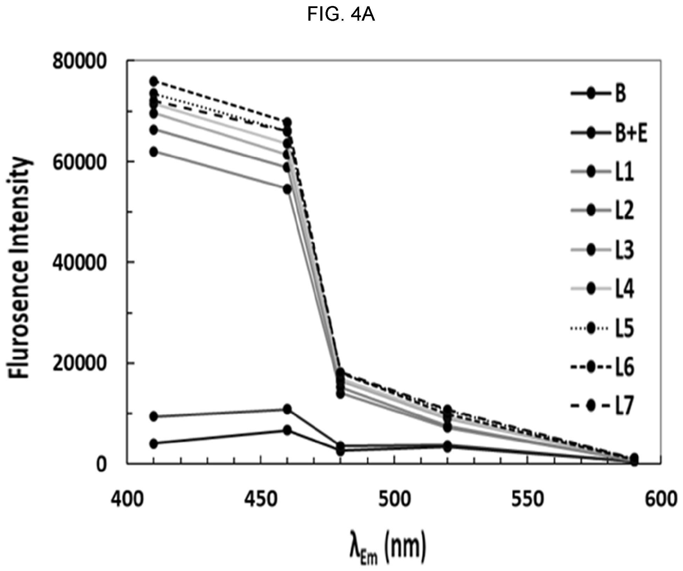

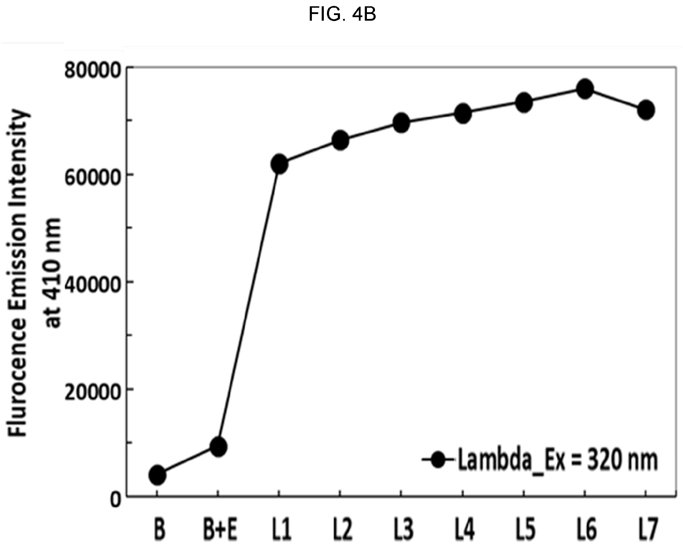

**Figure 4****:** Increase in fluorescence emission intensity after excitation at 320 nm for the samples (diluted to a protein concentration of 3.4 g/L) described in figure 3 **(A).** The increase in fluorescence emission intensity at 410 nm indicates the formation of oligo-tyrosine (e.g. di-tyrosine) cross-links **(B).** B: blank; B+E: blank + enzymes.

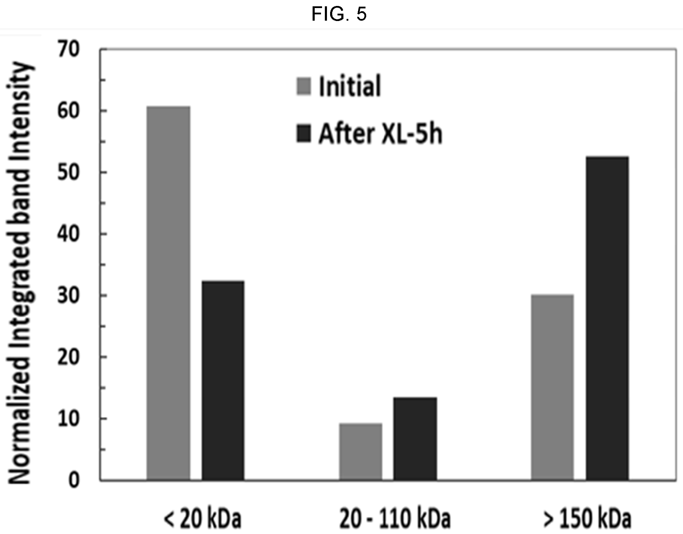

**Figure 5****:** Polymerization of sodium lignosulfonate due to covalent cross-linking induced by using a combination of lactose oxidase (LOX) and horseradish peroxidase (HRP). The reaction was controlled by sequential dosing of lactose every 30 minutes for up to 5 hours. The distribution of the small, medium and large polymers at the start and after 5 h of cross-linking are depicted.

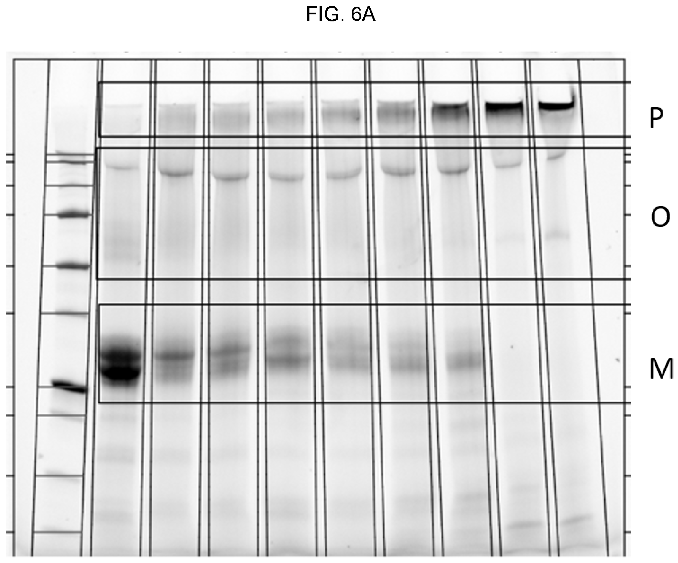

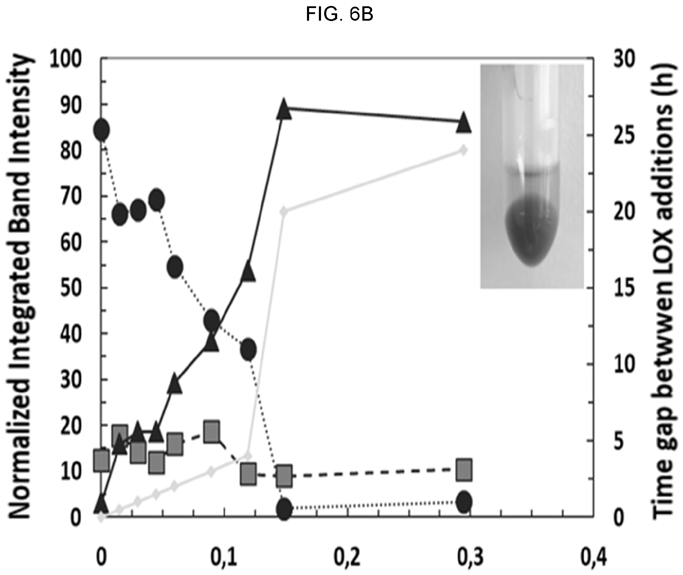

**Figure 6****:** Covalent coupling (conjugation/cross-linking) and polymerization of sodium caseinate and lignosulfonate due to covalent cross-linking induced by using a combination of lactose oxidase (LOX), lactose and horseradish peroxidase (HRP). The reaction was controlled by sequential dosing of LOX every 30 minutes and then the incubation (40 °C) was continued after 10 additions for up to 24 hours **(A).** P: polymers; O: oligomers; M: monomers. The distribution of the monomers, oligomers and large polymers at various extents of cross-linking are depicted **(B).** X axis shows cumulative LOX activity (U/mL).

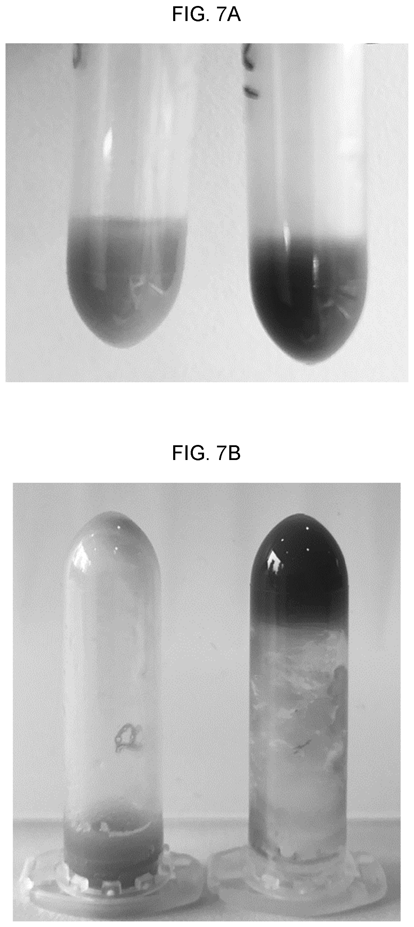

**Figure 7****:** Enhanced techno-functionality of the casein-lignosulfonate covalently conjugated polymers: Better film formation and wetting (spreading) on hydrophobic surface **(A)** (left: mixture of Na-caseinate and Na-lignosulfonate with in-situ formed CaP; right: Na-caseinate-lignosulfonate covalently conjugated polymers with in-situ formed CaP), and gelation of the conjugated polymer due to 'ionic bridges' formed by calcium phosphate particles **(B)** (left: mixture of Na-caseinate and Na-lignosulfonate with 2X in-situ formed CaP; right: Na-caseinate-lignosulfonate covalently conjugated polymers with 2X in-situ formed CaP).

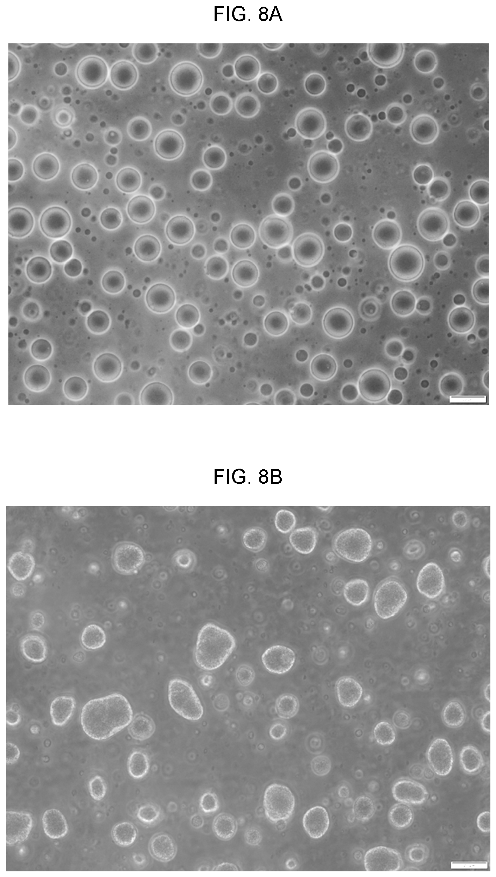

**Figure 8****:** Microcapsules formed by W/W phase separation (coacervation) of cross-linked Na-caseinate-lignosulfonate heteropolymers in alginate **(A).** The cells of *Lactobacillus animalis* encapsulated inside the microcapsules **(B).** The white bars at bottom right side indicate a length scale of 20 µm.

### DETAILED DESCRIPTION

### Bioactive agent

The methods disclosed herein involve encapsulation of a bioactive agent within heteropolymer particles.

The present methods are useful for encapsulating a variety of bioactive agents, in particular microorganisms. In one embodiment is thus provided a method for encapsulating a microorganism, said method comprising the steps of:
i) providing a microorganism, a heteropolymer obtained by cross-linking of a protein comprising at least one aromatic amino acid with a phenolic compound such as a polyphenolic compound, and a polymer, said polymer having the ability to phase separate from or with said heteropolymer, preferably having the ability to coacervate or to form a complex with said heteropolymer;
ii) contacting said microorganism with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said microorganism,
thereby obtaining a product comprising heteropolymer particles encapsulating said microorganism.

The microorganism may be a microorganism useful in the food, feed or pharmaceutical industry. In some embodiments, the microorganism is a bacterium, a probiotic bacterium, a probiotic microorganism, a spore forming bacterium or a lactic acid bacterium.

Combinations thereof can also be encapsulated in heteropolymer particles as described herein. In particular preferred embodiments, the microorganism is *Bifidobacterium bifidum, L. acidophilus, Lactobacillus delbruecki subsp. bulgaricus, Lactobacillus kefiranofaciens, and Lactobacillus helveticus, Lactobacillus animalis, Lactococcus lactis subsp. lactis* or members of the *Bacillus* genus such as B. *licheniformis, B. subtilis* or B. *paralicheniformis.*

In some embodiments, the bioactive agent is a plurality of bioactive agents. Hence, the present methods can be used to encapsulate several microorganisms.

The present methods may also be useful for encapsulating other bioactive agents, in particular phytochemicals, vitamins, minerals, peptides, bacteriocins, enzymes, nucleic acids and ion salts. In some embodiments, the bioactive agent is a phytochemical such as a carotenoid or a polyphenol, preferably curcumin or β-carotene. In some embodiments, the bioactive agent is a vitamin such as a water insoluble vitamin, preferably A vitamin, D vitamin, E vitamin or K vitamin. In some embodiments, the bioactive agent is a mineral. In some embodiments, the bioactive agent is a peptide. In some embodiments, the bioactive agent is a bacteriocin, such as a bacteriocin from a Gram-negative bacterium, preferably a microcin, a colicin or a tailocin; a bacteriocin from a Gram-negative bacterium such as a Class I bacteriocin, preferably nisin or a lantibiotic, a Class II bacteriocin, such as a Class IIa bacteriocin, a Class IIb bacteriocin, a Class Ilc bacteriocin, a Class IId bacteriocin or a Class IIe bacteriocin, a Class III bacteriocin, such as a subclass IIIa bacteriocin or a subclass Illb bacteriocin, or a Class IV bacteriocin, most preferably the bacteriocin is nisin. In some embodiments, the bioactive agent is an enzyme, such as lysozyme, lactase or a dairy enzyme such as chymosin. In some embodiments, the bioactive agent is a nucleic acid, such as DNA or RNA. In some embodiments, the bioactive agent is an ion or an ion salt, such as an iron salt, such as a zinc salt, such as a calcium salt, such as calcium phosphate, calcium carbonate, calcium sulphate or calcium oxalate.

### Heteropolymer

The present methods rely on the formation of heteropolymer particles, which encapsulate the bioactive agent.

The heteropolymer provided in the first step of the present methods is obtained by cross-linking a protein comprising at least one aromatic amino acid with a phenolic compound such as a polyphenolic compound. In some embodiments, the protein comprises at least one tyrosine.

The protein may be a milk protein such as a casein, whey protein, or the protein is a plant protein, a fish protein or an animal protein. The phenolic compound may be a plant phenolic compound, such as a phenolic compound from a grain such as a cereal, a bean such as a coffee bean, a leaf such as a tea leaf, a vegetable pulp or a vegetable peel such as from a tuberculous vegetable, or an animal phenolic compound, such as a phenolic compound from an insect, a mammal or a fish, such as a phenolic compound derived from side streams or waste streams from food or feed or paper or wood processing industry, such as a lignin, a lignosulfonate.

### FORMATION OF HETEROPOLYMER BY CROSS-LINKING

The heteropolymer is obtained by cross-linking a protein as described above, said protein comprising at least one aromatic amino acid, with a phenolic compound such as a polyphenolic compound, for example as described above.

Cross-linking may comprise the formation of intramolecular and/or intermolecular covalent cross-links between molecules of the phenolic compound. Cross-linking may also comprise the formation of intermolecular covalent cross-links between molecules of the phenolic compound and protein molecules. In particular, cross-linking may involve the formation of oligo-tyrosine cross-links, such as di-tyrosine cross-links and/or iso-di-tyrosine cross-links. These may be formed by covalent bonds of type C-C (e.g. in di-tyrosine cross-links). Other types of covalent bonds are C-O-C bonds, C-N bonds, S-S bonds and C-S bonds. C-O-C bonds can for example be in iso-di-tyrosine cross-links; C-N bonds can for example involve a carbon on the phenolic ring of the phenolic compound and a nitrogen on an amino chain of the protein. C-S bonds can for example involve a carbon on the phenolic ring of the phenolic compound and a sulfur on a sulphydryl side chain of the protein. S-S bonds can occur in the case of disulphide cross-links.

Cross-linking may be performed as is known in the art, for example non-enzymatically. In particular, the phenolic compound and the protein may be mixed and exposed to oxygen, as described e.g. in Strauss et al., 2004.

Cross-linking may also be performed enzymatically. In particular, useful enzymatic methods are described in patent application entitled "Method for producing modified food products" filed on the same date as the present application by the same applicant.

In particular, in some embodiments, the heteropolymer is obtained by a method comprising the steps of:
a) providing a protein comprising at least one aromatic amino acid, and a phenolic compound, as described herein above;
b) contacting said protein and said phenolic compound with a carbohydrate substrate such as lactose, a peroxidase (EC 1.11.1.7), and H₂O₂ or an oxidase selected from a cellobiose oxidase (EC 1.1.99.18) and a hexose oxidase such as a glucose oxidase (EC 1.1.3.4), and optionally a lactase;
c) incubating said protein and phenolic compound with said carbohydrate substrate, said peroxidase, and said H₂O₂ or said oxidase, whereby the oxidase catalyzes conversion of the carbohydrate substrate to a corresponding organic acid and H₂O₂ in the presence of oxygen, whereby the peroxidase catalyzes cross-linking of said phenolic compound using said H₂O₂ as a co-substrate to obtain heteropolymers of said phenolic compound and said protein.

Accordingly, in some embodiments the method for encapsulating a microorganism comprises the steps of:
i) providing a microorganism, a heteropolymer obtained by cross-linking of a protein comprising at least one aromatic amino acid with a phenolic compound, and a polymer, said polymer having the ability to phase separate from or with said heteropolymer, preferably having the ability to coacervate or to form a complex with said heteropolymer;
ii) contacting said microorganism with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said microorganism,

thereby obtaining a product comprising heteropolymer particles encapsulating said microorganism,
wherein the heteropolymer is obtained by a method comprising the steps of:
   a) providing a protein comprising at least one aromatic amino acid, and a phenolic compound, as described herein above;
   b) contacting said protein and said phenolic compound with a carbohydrate substrate such as lactose, a peroxidase (EC 1.11.1.7), and H₂O₂,
   c) incubating said protein and phenolic compound with said carbohydrate substrate, peroxidase, and said H₂O₂, whereby the peroxidase catalyzes cross-linking of said phenolic compound using said H₂O₂ as a co-substrate to obtain heteropolymers of said phenolic compound and said protein.

In other embodiments the method for encapsulating a microorganism comprises the steps of:
i) providing a microorganism, a heteropolymer obtained by cross-linking of a protein comprising at least one aromatic amino acid with a phenolic compound, and a polymer, said polymer having the ability to phase separate from or with said heteropolymer, preferably having the ability to coacervate or to form a complex with said heteropolymer;
ii) contacting said microorganism with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said microorganism,

thereby obtaining a product comprising heteropolymer particles encapsulating said microorganism,
wherein the heteropolymer is obtained by a method comprising the steps of:
   a) providing a protein comprising at least one aromatic amino acid, and a phenolic compound, as described herein above;
   b) contacting said protein and said phenolic compound with said carbohydrate substrate, a peroxidase (EC 1.11.1.7), and an oxidase selected from a cellobiose oxidase (EC 1.1.99.18) and a hexose oxidase such as a glucose oxidase (EC 1.1.3.4);
   c) incubating said protein and phenolic compound with said carbohydrate substrate, said peroxidase, and said oxidase, whereby the oxidase catalyzes conversion of the carbohydrate substrate to a corresponding organic acid and H₂O₂ in the presence of oxygen, whereby the peroxidase catalyzes cross-linking of said phenolic compound using said H₂O₂ as a co-substrate to obtain heteropolymers of said phenolic compound and said protein.

The formation of heteropolymer can thus rely on the addition of external H₂O₂ or H₂O₂ can be formed *in situ* by the action of the oxidase converting the carbohydrate substrate to a corresponding organic acid and H₂O₂. In preferred embodiments, the carbohydrate substrate is lactose and the acid is lactobionic acid. The peroxidase can then catalyze cross-linking of the phenolic compound using said H₂O₂ as a co-substrate to obtain heteropolymers of the phenolic compound and of the protein.

The formation of heteropolymer may be carried out with oxygen sparging, *in-situ* oxygen generation, forced oxidation or enzymatic oxidation, as described for example in Strauss et al., 2014.

Steps i), ii) and iii) above may be as described herein. The heteropolymer formation may be performed simultaneously with step iii), in particular using coacervation.

Oxygen may be required for the action of the oxidase. The substrate comprising a carbohydrate substrate may therefore also comprises oxygen. The oxygen may be naturally present in the substrate, or it may be added as is known in the art.

The carbohydrate substrate may be any carbohydrate which can be converted into a corresponding organic acid and H₂O₂ by the action of the oxidase, which is a cellobiose oxidase or a hexose oxidase such as a glucose oxidase as described herein in detail. The carbohydrate substrate may thus be lactose, which can be converted to lactobionic acid and H₂O₂ by the action of the oxidase.

In some embodiments, the carbohydrate substrate is a hexose, which can for example be obtained by treating the substrate prior to contacting it with the appropriate hexose oxidase. For example, the substrate comprises lactose, and is treated with lactase, thereby yielding glucose and galactose. A glucose oxidase can then convert the galactose and/or the glucose into galactonic acid and gluconic acid, and H₂O₂, in the presence of oxygen. In another embodiment, the carbohydrate substrate is glucose, which can be converted to gluconic acid and H₂O₂ by the action of the oxidase. In another embodiment, the carbohydrate substrate is galactose, which can be converted to galactonic acid and H₂O₂ by the action of the oxidase. In another embodiment, the carbohydrate substrate is maltose, which can be converted to maltobionic acid and H₂O₂ by the action of the oxidase. In another embodiment, the carbohydrate substrate is xylose, which can be converted to xylonic acid and H₂O₂ by the action of the oxidase. In another embodiment, the carbohydrate substrate is cellobiose, which can be converted to cellobionic acid and H₂O₂ by the action of the oxidase. In another embodiment, the carbohydrate substrate is mannose, which can be converted to mannonic acid and H₂O₂ by the action of the oxidase. In another embodiment, the carbohydrate substrate is fructose, which can be converted to fructonic acid and H₂O₂ by the action of the oxidase.

It is to be understood throughout the present disclosure that the carbohydrate substrate on which the oxidase acts may be inherently present in the product to be modified, i.e. the substrate, or it may be obtained by treating the substrate as is known in the art. For example, if the substrate is a dairy product, the substrate may be treated with lactase, whereby the lactose present in the substrate is converted to galactose and glucose, which are converted by the oxidase to galactonic acid and gluconic acid, respectively, while generating H₂O₂ in the substrate. Such additional enzymatic treatment may occur prior to step i) or concomitantly with any of steps i), ii) and iii). Preferably, such treatment is performed prior to or concomitantly with step i). In embodiments where a hexose oxidase is used, the protein and the phenolic compound are preferably contacted with the carbohydrate substrate, a peroxidase and H₂O₂ and with a lactase.

The formation of heteropolymers thus relies on *in situ* formation of H₂O₂ by the action of an oxidase selected from a cellobiose oxidase and a hexose oxidase such as a glucose oxidase, which converts the carbohydrate substrate to a corresponding organic acid and H₂O₂. The peroxidase can then catalyze cross-linking of the first compound using said H₂O₂ as a co-substrate to obtain a cross-linked compound.

In some embodiments, the oxidase is a cellobiose oxidase. Cellobiose oxidase is an unspecific enzyme of EC number EC 1.1.99.18, capable of catalyzing conversion of different carbohydrate substrates into the corresponding acids and H₂O₂. The enzyme is unspecific, and can convert for example:
- Lactose to lactobionic acid,
- Glucose to gluconic acid,
- Galactose to galactonic acid,
- Maltose to maltobionic acid,
- Xylose to xylonic acid,
- Cellobiose to cellobionic acid,
- Mannose to mannonic acid,
- Fructose to fructonic acid,
while generating H₂O₂.

Cellobiose oxidase (EC 1.1.99.18) may alternatively be termed lactose oxidase (LOX) or carbohydrate oxidase, and the terms will be used interchangeably herein.

In some embodiments, the cellobiose oxidase is LactoYield^{®} (Chr. Hansen A/S). In some embodiments, the cellobiose oxidase (EC 1.1.99.18) enzyme is an enzyme:
(i): comprising the polypeptide sequence of position 23-495 of SEQ ID NO: 2 of EP 1 041 890 B1, which starts with Gly in position 23 and ends with Lys in position 495; or
(ii): a variant of (i), wherein the variant comprises less than 20 amino acid alterations, preferably less than 10 amino acid alterations, more preferably less than 5 amino acid alterations amino acid alterations (preferably a substitution, a deletion or an insertion - most preferably a substitution) as compared to polypeptide sequence of (i).

Useful cellobiose oxidases are described in application "Use of cellobiose oxidase for reduction of reduction of Maillard reaction" filed by same applicant on May 24, 2018.

The cellobiose oxidase may also or alternatively naturally be present in the substrate.

In other embodiments, the oxidase is a hexose oxidase such as a glucose oxidase (EC 1.1.3.4), which can catalyze the conversion of a hexose such as glucose to the corresponding organic acid such as glucobionic acid and H₂O₂.

In some embodiments of the method, the concentration of oxidase, i.e. the cellobiose oxidase or the glucose oxidase, relative to the substrate is in the range of 0.0001 to 15 U/g substrate, such as 0.01 U/g substrate, 0.05 U/g substrate, or 0.15 U/g substrate, for example between 0.001 and 12.5 U/g substrate, such as between 0.005 and 10 U/g substrate, for example between 0.01 and 7.5 U/g substrate, such as between 0.05 and 5 U/g substrate, for example between 0.1 and 2.5 U/g substrate, such as between 0.15 and 1 U/g substrate, for example between 0.25 and 0.75 U/g substrate, such as 0.5 U/g substrate.

Accordingly, in some embodiments of the method where the substrate is a dairy product, the concentration of oxidase, e.g. the cellobiose oxidase or glucose oxidase, relative to the dairy product is in the range of 0.0001 to 15 U/g dairy product, such as 0.01 U/g dairy product, 0.05 U/g dairy product, or 0.15 U/g dairy product, for example between 0.001 and 12.5 U/g dairy product, such as between 0.005 and 10 U/g dairy product, for example between 0.01 and 7.5 U/g dairy product, such as between 0.05 and 5 U/g dairy product, for example between 0.1 and 2.5 U/g dairy product, such as between 0.15 and 1 U/g dairy product, for example between 0.25 and 0.75 U/g substrate, such as 0.5 U/g dairy product. The dairy product may be as described above, i.e. a yogurt, quark, a cheese such as a soft cheese, a drinking yogurt, a cheese spread, skyr or milk, such as soy milk, sheep milk, goat milk, buffalo milk, yak milk, lama milk, camel milk or cow milk, or a combination thereof, optionally supplemented with plant material.

In some embodiments of the method, the oxidase is a cellobiose oxidase, such as LactoYield^{®}, and the concentration of cellobiose oxidase, e.g. the LactoYield^{®} cellobiose oxidase, relative to the substrate is in the range of 0.0001 to 15 U/g substrate, such as 0.01 U/g substrate, 0.05 U/g substrate, or 0.15 U/g substrate, for example between 0.001 and 12.5 U/g substrate, such as between 0.005 and 10 U/g substrate, for example between 0.01 and 7.5 U/g substrate, such as between 0.05 and 5 U/g substrate, for example between 0.1 and 2.5 U/g substrate, such as between 0.15 and 1 U/g substrate, for example between 0.25 and 0.75 U/g substrate, such as 0.5 U/g substrate.

Accordingly, in some embodiments of the method where the substrate is a dairy product, the concentration of cellobiose oxidase, e.g. the LactoYield^{®} cellobiose oxidase, relative to the dairy product is in the range of 0.0001 to 15 U/g dairy product, such as 0.01 U/g dairy product, 0.05 U/g dairy product, or 0.15 U/g dairy product, for example between 0.001 and 12.5 U/g dairy product, such as between 0.005 and 10 U/g dairy product, for example between 0.01 and 7.5 U/g dairy product, such as between 0.05 and 5 U/g dairy product, for example between 0.1 and 2.5 U/g dairy product, such as between 0.15 and 1 U/g dairy product, for example between 0.25 and 0.75 U/g substrate, such as 0.5 U/g dairy product. The dairy product may be as described above, i.e. a yogurt, quark, a cheese such as a soft cheese, a drinking yogurt, a cheese spread, skyr or milk, such as soy milk, sheep milk, goat milk, buffalo milk, yak milk, lama milk, camel milk or cow milk, or a combination thereof, optionally supplemented with plant material.

The phenolic compound may be a plant phenolic compound, such as a phenolic compound from a grain such as a cereal, a bean such as a coffee bean, a leaf such as a tea leaf, a vegetable pulp or a vegetable peel such as from a tuberculous vegetable, or an animal phenolic compound, such as a phenolic compound from an insect, a mammal or a fish, such as a phenolic compound derived from side streams or waste streams from food or feed or paper or wood processing industry, such as a lignin or a lignosulfonate. In particular, the phenolic compound may be lignin, lignosulfonate, caffeic acid, cholorogenic acid, a flavonoid, a flavonol, quercetin, rutin, tannic acid, vanillin, p-coumaric acid, ferulic acid or ABTS.

The protein may be a milk protein such as a casein or whey protein, or the protein may be a plant protein, a fish protein or an animal protein.

In particular embodiments, the protein is comprised within a first substrate and the phenolic compound is comprised within a second substrate. In some embodiments, the protein and the phenolic compound are comprised within the same substrate, i.e. the first and the second substrate are one same substrate.

In some embodiments, the second substrate comprises in the range of 0.01% to 30% w/w of phenolic compound, such as 0.05%, 1%, 5%, 10%, 15%, 20%, 25% w/w, for example between 2.5 and 6% w/w, such as 3.5% w/w.

In some embodiments, step c) is performed at a temperature of 4°C to 75°C, such as between 4°C and 72°C, for example between 4°C and 70°C, such as between 4°C and 65°C, for example between 4°C and 60°C, such as between 4°C and 55°C, for example between 4°C and 50°C, such as between 4°C and 45°C, for example between 4°C and 40°C, such as between 4°C and 37°C, for example between 4°C and 35°C, such as between 4°C and 30°C, for example between 4°C and 25°C, such as between 4°C and 20°C, for example between 4°C and 15°C, such as between 4°C and 10°C, or such as between 10°C and 75°C, for example between 15°C and 75°C, such as between 20°C and 75°C, for example between 25°C and 75°C, such as between 30°C and 75°C, for example between 35°C and 75°C, such as between 37°C and 75°C, for example between 40°C and 75°C, such as between 45°C and 75°C, for example between 50°C and 75°C, such as between 55°C and 75°C, for example between 60°C and 75°C, such as between 65°C and 75°C, for example between 72°C and 75°C, such as at 75°C, 72°C, 40°C, 37°C, 25°C or 4°C.

In some embodiments, step c) is performed for a duration of between 15 seconds and 144 hours, such as between 30 seconds and 132 hours, for example between 1 minute and 120 hours, such as between 2 minutes and 108 hours, for example between 5 minutes and 96 hours, such as between 10 minutes and 84 hours, for example between 20 minutes and 72 hours, such as between 30 minutes and 60 hours, for example between 1 hour and 48 hours, such as between 2 hours and 44 hours, for example between 3 hours and 40 hours, such as between 3 hours and 36 hours, for example between 4 hours and 32 hours, such as between 4 hours and 28 hours, for example between 5 hours and 24 hours, such as between 5 hours and 20 hours, for example between 6 hours and 16 hours, such as between 6 hours and 12 hours, for example between 1 hour and 10 hours, such as between 2 hours and 8 hours, for example between 3 hours and 6 hours, such as 3, 4, 5 or 6 hours.

In some embodiments, the pH of the substrate in any of steps a), b) or c) is in the range of 1 to 12, such as between 3.5 to 8.5, such as between 4.0 and 8.0, for example between 4.5 and 7.5, such as between 5.0 and 7.2, for example between 5.5 and 7.0, such as between 6.0 and 6.9, for example between 6.2 and 6.8, such as between 6.4 and 6.7, for example 6.6.

In some embodiments, step c) is performed at a temperature of 75°C for 15 seconds, or at a temperature of 72°C for 30 seconds, or at a temperature of 40°C for 3 to 6 hours, such as at a temperature of 40°C for 3 hours, for 4 hours, for 5 hours or for 6 hours.

Reference is made to application "Method for producing modified food products" filed on the same date and by the same applicant, which describes in detail how to form heteropolymers by cross-linking.

The carbohydrate substrate such as lactose contacted with the protein and the phenolic compound in step b) may be added to the mixture; however, in some embodiments, the carbohydrate substrate, for example lactose, is present in the first and/or the second substrate, for example if the substrate is milk or a dairy product. In some embodiments, the first and/or second substrate is a yogurt, quark, a cheese such as a soft cheese, a drinking yogurt, a cheese spread, skyr or milk, such as soy milk, sheep milk, goat milk, buffalo milk, yak milk, lama milk, camel milk or cow milk, or a combination thereof, optionally supplemented with plant material.

In some embodiments, the oxidase selected from a lactose oxidase and a hexose oxidase such as a glucose oxidase is provided in the range of 0.0001 to 30 U/g, such as 0.01 U/g, 0.05 U/g, or 0.15 U/g, for example between 0.001 and 12.5 U/g, such as between 0.005 and 10 U/g, for example between 0.01 and 7.5 U/g, such as between 0.05 and 5 U/g, for example between 0.1 and 2.5 U/g, such as between 0.15 and 1 U/g, for example between 0.25 and 0.75 U/g, such as 0.5 U/g.

The peroxidase can be exogenous, i.e. it is added to the other compounds exogenously, or it can be endogenous, e.g. it can be present in the first and/or the second substrate.

In some embodiments, the peroxidase is a lactoperoxidase. In some embodiments, the peroxidase is a horseradish peroxidase (HRP). In some embodiments, the lactoperoxidase is a lignin peroxidase. In some embodiments, the peroxidase is a Coprinus peroxidase. In some embodiments, the peroxidase is myeloperoxidase.

In some embodiments, the concentration of peroxidase is in the range of 0.001 to 5000 U/g, such as 5, 15, 30, or 50 U/g, for example between 0.01 and 250 U/g, such as between 0.05 and 125 U/g, for example between 0.1 and 100 U/g, such as between 0.5 and 75 U/g, for example between 1 and 50 U/g, such as between 5 and 40 U/g, for example between 10 and 30 U/g, for example 15, 20 or 25 U/g.

In some embodiments, the heteropolymer is a Na-casein-lignosulfonate heteropolymer.

Using said methods for forming heteropolymer by cross-linking, the heteropolymer may have an averaged degree of polymerization (DP) from 2 to 100000, such as from 3 to 100000, such as from 5 to 1000, such as from 8 to 200, such as from 9 to 150, such as 100 or 125.

### Phase separation

In step i) of the present methods, a bioactive agent and a heteropolymer as described herein are provided. Also provided in step i) is a polymer having the ability to phase separate from (coacervation or segregative phase separation) or with (complex coacervation or associative phase separation) the heteropolymer. The bioactive agent and the heteropolymer are in step ii) contacted with one another. In step iii) phase separation of the heteropolymer from or with the polymer is induced, resulting in a continuous phase and a dispersed phase, wherein one of the phases comprises heteropolymer particles encapsulating the bioactive agent.

Phase separation can advantageously be used to encapsulate molecules, such as the bioactive agents described herein, and in particular sensitive molecules, for example molecules sensitive to external stress factors such as oxygen, humidity, heat or light. Phase separation requires:
- a molecule to be encapsulated; this can be any of the bioactive agents described herein, and can be hydrophobic or hydrophilic;
- a first compound which can bind to the molecule to be encapsulated; this compound may be in a solid or liquid form, and can be hydrophobic or hydrophilic;
- a second compound which can phase separate from or with the first compound under specific physico-chemical conditions.

Examples of first compounds include: calcium phosphate coated with a phospho-peptide; oil; di-block polymers with hydrophobic and hydrophilic blocks dissolved in water; polymers dissolved in water, for example positively charged polymers. Examples of second compounds include: water; water and an emulsifier; polymer dissolved in water, for example a hydrophilic or a charged polymer, such as a negatively charged polymer.

Specific examples of combinations include the following. An enzyme, such as lactase, calcium phosphate coated with a phospho-peptide, and water. When the calcium ion and the phosphate ion are present above certain concentrations at pH > 7, they can phase separate (here precipitate) out of the water (acting as solvent), whereby the enzyme is entrapped inside the porous inorganic particles.

Another combination is beta-carotene with oil, water and an emulsifier. Beta-carotene is hydrophobic and dissolves only in the oil phase, which separates from the water phase to form an oil in water emulsion. The emulsion drops are stabilized by the emulsifier present in the water phase and encapsulate the beta-carotene.

Another combination is a bacterium, for example a probiotic bacterium, a di-block polymer dissolved in water, and a hydrophilic or charged polymer dissolved in water. The bacterium binds to the di-block polymer by e.g. patchy hydrophobic interactions and/or a combination of other interactions such as van der Waals interactions, hydrogen bonding, electrostatic interactions inter alia. Above a certain concentration, the di-block polymer and the polymer dissolved in water phase separate (coacervate), and the bacterium is encapsulated in droplets of the di-block polymer.

Another combination is a bacterium, for example a probiotic bacterium, a positively charged polymer dissolved in water and a negatively charged polymer dissolved in water. The polymers form an electrostatic complex which phase separates from the water in a process of complex coacervation. The bacteria are entrapped in the complex by patchy charge interactions and/or a combination of other interactions such as van der Waals interactions, hydrogen bonding, hydrophobic interactions inter alia.

The person of skill in the art knows how to induce or trigger phase separation. Some useful physico-chemical parameters that can be used to do so are temperature, pressure, solubility, ionic strength, pH and concentration of the different compounds.

Emulsion technology using suitable emulsifiers (surfactants) can be advantageously used for obtaining encapsulated microorganisms to be incorporated in liquid foods. Oil in water emulsions are typically used for encapsulating hydrophobic compounds, while water in oil emulsions are typically used for encapsulating hydrophilic compounds. The emulsification can be followed by a drying step, to obtain a dry product comprising particles encapsulating the bioactive agent, e.g. a powder. The drying step may be a step of freeze-drying as is known in the art.

In preferred embodiments where the bioactive agent is a cell and a dry product such as a powder is obtained, at least 1.10⁵ cells may be encapsulated per g of dry product, such as at least 1.10⁶, 1.10⁷, 1.10⁸, 1.10⁹, 1.10¹⁰, 1.10¹¹ cells per g of dry product, or more. In other embodiments where the bioactive agent is not a cell, at least 1.10⁸ molecules of the bioactive agent may be encapsulated per g of dry product, such as at least 1.10⁹, 1.10¹⁰, 1.10¹¹, 1.10¹², 1.10¹³, 1.10¹⁴, 1.10¹⁵, 1.10¹⁶, 1.10¹⁷, 1.10¹⁸, 1.10¹⁹, 1.10²⁰ molecules of bioactive agent per g of dry product, or more.

Coacervation refers to liquid-liquid phase separation, mainly resulting from segregation of molecules or association of oppositely charged molecules (such as macro-ions, polyelectrolytes, polysaccharides, protein etc.) or from hydrophobic molecules/ proteins. When coacervation happens, two liquid phases are formed: a coacervate phase comprising coacervate droplets and a dilute phase. The coacervate phase corresponds to the dispersed phase of an emulsion. The terms "coacervate phase" and "dispersed phase" will thus be used herein interchangeably. The dilute phase corresponds to the continuous phase of an emulsion. The terms "dilute phase" and "continuous phase" will thus be used herein interchangeably.

In some embodiments the phase separation induced in step iii) is coacervation or complex coacervation.

In some embodiments, the polymer is an alginate, a xyloglucan, a polymerized casein glycomacropeptide, a chitosan, a starch, a modified starch, a food gum, a food stabilizer or a food hydrocolloid. Preferably the polymer is an alginate.

Accordingly, in step iii), a dispersed or coacervate phase is obtained and a continuous or dilute phase is obtained. The heteropolymer particles encapsulating the bioactive agent formed during the phase separation are comprised within one of the phases. In some embodiments, the heteropolymer particles are within the dispersed phase. In other embodiments, the heteropolymer particles are within the continuous phase. Preferably, the heteropolymer particles are within the dispersed phase.

With the present methods, at least 0.001% of the bioactive agent is encapsulated, such as at least 0.01%, such as at least 0.1%, such as at least 1%, such as at least 2%, such as at least 3%, such as at least 4%, such as at least 5%, at least 10%, at least 15%, at least 20%, at least 25%, at least 30%, at least 35%, at least 40%, at least 45%, at least 50%, at least 55%, at least 60%, at least 65%, at least 70%, at least 99% or more.

### Optional steps

In some embodiments, the method further comprises one or more optional steps, as detailed below.

In some embodiments, the method further comprises inducing ionic cross-linking of said heteropolymer with a multivalent ion such as a calcium ion before or after step iii). In some embodiments, the multivalent is the bioactive agent to be encapsulated.

The method may alternatively or additionally comprise further cross-linking said heteropolymer and/or polymer by:
- contacting said heteropolymer and/or polymer with an enzyme such as a microbial transglutaminase, a polyphenol oxidase, a tyrosinase, a laccase, and/or
- contacting said polymer with an enzyme such as a microbial transglutaminase, a polyphenol oxidase, a tyrosinase, a laccase, or with a reagent such as genipin, before formation of said heteropolymer, and/or
- contacting said heteropolymer and/or polymer with a reagent such as genipin or glutaraldehyde.

The method may alternatively or additionally comprise a step of pasteurization or a step of sterilization of any one or all of the bioactive agent, the heteropolymer, the protein, the phenolic compound or the polymer, prior to encapsulation of said bioactive agent. **In** embodiments where it is desirable that the bioactive agent be maintained viable, for example if the bioactive agent is a microorganism, particularly a probiotic bacterium, the bioactive agent is not submitted to the step of pasteurization or sterilization.

The method may alternatively or additionally comprise the step of providing an antioxidant such as ascorbate and/or a cryoprotectant such as a disaccharide, preferably trehalose, in step i) or ii), wherein the antioxidant and/or the cryoprotectant are comprised in the same phase as the heteropolymer particles after step iii). Addition of an antioxidant and/or cryoprotectant may be desirable to further protect the bioactive agent after encapsulation, in particular where the bioactive agent is a microorganism such as a probiotic bacterium.

The method may alternatively or additionally comprise the step of freezing and the step of drying such as freeze drying, and/or the step of spray drying after step iii). Freezing in liquid nitrogen and freeze drying may be desirable to further protect the bioactive agent after encapsulation, in particular where the bioactive agent is a microorganism such as a probiotic bacterium.

In addition, the present methods may be combined with other encapsulation, microencapsulation or entrapment processes as known in the art, e.g. emulsification processes, sol-gel processes, gelation, microgel formation and spray coating in a fluidized bed.

The product obtained in or after step iii), which comprises heteropolymer particles encapsulating the bioactive agent, may further be processed in a food product, a feed product or a pharmaceutical product, as is known in the art.

### Modified food, feed, cosmetic, plant health, seed health or pharmaceutical product

The present methods are useful for making a modified food, feed, cosmetic or pharmaceutical product comprising heteropolymer particles encapsulating a bioactive agent such as a microorganism. The methods are also useful for obtaining plant health products or seed health products for improving plant health or seed health.

Accordingly, herein is also provided methods for making a modified food, feed, cosmetic, plant health, seed health or pharmaceutical product comprising heteropolymer particles encapsulating a bioactive agent such as a microorganism, said methods comprising the steps of:
i) providing a bioactive agent such as a microorganism, a heteropolymer obtained by cross-linking of a protein comprising at least one aromatic amino acid with a phenolic compound such as a polyphenolic compound, and a polymer such as a biopolymer, said polymer having the ability to phase separate from or with said heteropolymer, preferably having the ability to coacervate or to form a complex with said heteropolymer;
ii) contacting said bioactive agent with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent,
   wherein
   - if the protein is provided in a first substrate and the phenolic compound is provided in a second substrate, one of the first and second substrate is the food to be modified, steps ii) and iii) are performed within said first or second substrate, and the modified food, feed, cosmetic, plant health, seed health or pharmaceutical product is obtained in step iii); or
   - if the protein and the phenolic compound are provided in a composition, steps ii) and iii) are performed within said composition, a composition comprising heteropolymers encapsulating said bioactive agent is obtained in step iii), and the method further comprises a step of:
      iv) adding the composition comprising heteropolymer particles encapsulating said bioactive agent to a food, feed, cosmetic, plant health, seed health or pharmaceutical product to be modified;
thereby obtaining a modified food, feed, cosmetic, plant health, seed health or pharmaceutical product comprising heteropolymer particles encapsulating said bioactive agent.

The bioactive agent, the microorganism, the heteropolymer, the phenolic compound, the polymer, the phase separation and/or the heteropolymer particles may be as described herein. Steps i), ii) and iii) may be as described herein above. **In** addition, the method may comprise any of the optional steps described in the section "optional steps".

The food or feed product may be a dairy product, such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material. The product may be a fermented milk product.

Relevant pharmaceutical products may be a medicament, a tooth paste or a bone cement.

### Composition comprising heteropolymer particles encapsulating a bioactive agent

Also provided herein is a composition comprising heteropolymer particles of a phenolic compound and a protein, and a bioactive agent encapsulated therein, obtainable or obtained by the methods described herein.

Accordingly, is provided herein a composition comprising heteropolymer particles of a phenolic compound and a protein, and a bioactive agent encapsulated therein, obtainable or obtained by a method comprising the steps of:
i) providing a microorganism, a heteropolymer obtained by cross-linking of a protein comprising at least one aromatic amino acid with a phenolic compound such as a polyphenolic compound, and a polymer, said polymer having the ability to phase separate from or with said heteropolymer, preferably having the ability to coacervate or to form a complex with said heteropolymer;
ii) contacting said microorganism with said heteropolymer,
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said microorganism,

thereby obtaining a product comprising heteropolymer particles encapsulating said microorganism,
wherein the composition comprises or consists of the product.

Alternatively, the heteropolymer particles may be recovered from the product and added to the composition as is known in the art.

The composition may be a food composition, a feed composition or a pharmaceutical composition. The composition may be obtained by methods known in the art, which can be used to formulate the heteropolymer particles in a composition. This can be particularly relevant for liquid feeds or foods.

The bioactive agent, the microorganism, the heteropolymer, the phenolic compound, the polymer, the phase separation and/or the heteropolymer particles may be as described herein. Steps i), ii) and iii) may be as described herein above. **In** addition, the method may comprise any of the optional steps described in the section "optional steps".

The food or feed composition may be a dairy product, such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material. The food or feed composition may be a fermented milk product.

Relevant pharmaceutical compositions may be a medicament, a tooth paste or a bone cement.

### Method for delivering a bioactive agent to a subject

Also provided herein is a method for delivering a bioactive agent to a subject, said method comprising administering to the subject the modified food, feed, cosmetic, plant health, seed health or pharmaceutical product obtained by the methods disclosed herein. The methods for delivering a bioactive agent may thus comprise all the steps described herein above relating to the encapsulation of a bioactive agent in heteropolymer particles, and a step of administering the heteropolymer particles to a subject. The heteropolymer particles encapsulating the bioactive agent may be comprised in a modified food, feed, cosmetic, plant health, seed health or pharmaceutical product as described herein.

The modified food, feed or pharmaceutical products obtainable by the present methods can thus be administered to a subject to deliver the bioactive agent.

The subject may be a mammal, such as a human. The subject may be an animal, particularly an animal of the food industry, e.g. a pig, a cattle animal, a poultry animal, such as a chicken.

The bioactive agent, the microorganism, the heteropolymer, the phenolic compound, the polymer, the phase separation and/or the heteropolymer particles may be as described herein. Steps i), ii) and iii) may be as described herein above. **In** addition, the method may comprise any of the optional steps described in the section "optional steps".

In some embodiments, the subject is a plant. The modified products obtainable by the present methods may also be useful to improve plant health, for example by administering to the plant by methods known in the art encapsulated bioactive agents which can improve plant health and/or longevity and/or growth, for example encapsulated microorganisms or spores such as *Bacillus* spores can be administered to the plant. Such products are termed herein "plant health products" and refer to products which increase the health of a plant. The modified product may also be a seed health product, which can improve seed health, and can be used to coat plant seeds.

### EXAMPLES

### Example 1: Materials

Sodium dihydrogen phosphate monohydrate, di-sodium hydrogen phosphate dihydrate and lactose monohydrate of analytical grade were from Merck. Sodium lignosulfonate was procured from Borregaard (DP-3352). The sodium caseinate was taken from the bulk used in Chr. Hansen Natural Colors A/S (Lot# 500459/0005092876). The calcium chloride concentrate (50 % w/v) was the one used for preparing clotting model milk at Chr. Hansen (Batch# 412, density = 1.36 g/mL). The lactose oxidase (LOX) used was the formulated product sold by Chr. Hansen (LactoYield^{®}, activity = 15 LOX U/g). The horseradish peroxidase (HRP) was from Sigma Aldrich (P8125, activity = 50 kU/g, where 1-unit forms 1 mg purpurogallin from pyrogallol in 20 s at pH 6.0 at 20 °C).

### Example 2: Methods

### Solution preparation

The details of the solution volumes and concentrations of various substrates and enzymes are given in table 1. The substrates were dissolved in sodium phosphate buffer (200 mM, pH 7.0). In the case of sodium caseinate, the weighed amount of powder was suspended in 75 % of the final volume and the tube was left rotating (inverting) overnight at room temperature for complete solubilization. Next day, buffer was added to make the volume up to the final volume.

**Table 1. Details of cross-linking reactions**

| | Na-caseinate XL | Na-lignosulfonate XL | Na-caseinate-lignosulfonate XL |
|---|---|---|---|
| Solvent | 0.2 M NaP buffer, pH 7.0, volume 1 mL | 0.2 M NaP buffer, pH 7.0, volume 1 mL | 0.2 M NaP buffer, pH 7.0, volume 10 mL |
| Skimmed milk powder (SMP) | - | - | - |
| Na-caseinate | 80 g/L | - | 40 g/L |
| Na-lignosulfonate (DP-3352) | - | 30 g/L | 15 g/L |
| Horseradish peroxidase (HRP) | 100 U/mL | 100 U/mL | 100 U/mL |
| LOX | 0.15 U/mL | 0.15 U/mL | 0.15 U/mL |
| Sequential LOX addition | - | - | Stock: 15 U/mL |
| | | | 10 µL added per step |
| | | | 10 steps at 30 min. intervals |
| Sequential lactose addition | Stock: 180 g/L lactose | Stock: 180 g/L lactose | - |
| | 10 µL added per step | 10 µL added per step | |
| | 7 steps at 30 min. intervals | 7 steps at 30 min. intervals | |
| | Total lactose: 10.7 g/L | Total lactose: 10.7 g/L | |
| lactose | - | - | 50 g/L |
| Temperature (°C) | 40 | 40 | 40 |
| Enzyme inactivation | 90°C, 10 min | 90°C, 10 min | 90°C, 10 min |

### Enzymatic cross-linking

For cross-linking Na-caseinate or Na-lignosulfonate, HRP and LOX were added to the substrate solutions, followed by pre-incubation at 40 °C for 15 minutes in the thermomixer (Eppendorf). The cross-linking (XL) reaction in these systems was started and controlled by sequential dosing of lactose stock solution as described in **table 1.** After each lactose addition step, the XL reaction was continued for 30 minutes at 40°C. The reaction was stopped after 30 minutes by heating the Eppendorf tube at 90°C for 10 minutes, followed by cooling down to 4°C. Similarly, the samples of various extents of cross-linking were collected after each lactose addition step and a gap of 30 minutes. 50 µL of each time point sample was diluted with 950 µL of MQ-water and stored at 4°C till further analysis.

Covalently conjugated block polymers of Na-caseinate-lignosulfonate were made mixing both substrates at a fixed molar ratio and then cross-linking them using HRP and LOX. The XL reaction in this case was controlled by sequential dosage of LOX, while a fixed amount of lactose was added to the substrate solution. After addition of lactose and HRP, the solution was pre-incubation at 40°C for 15 minutes in the thermomixer (Eppendorf). The cross-linking reaction was started and controlled by sequential dosing of LOX stock solution as described in table 1. After each LOX addition step, the XL reaction was continued for 30 minutes at 40°C.

At the end of 30 minutes, 50 µL of the sample was taken out and added to 950 µL of MQ-water kept at 90°C and then the heating was continued for 10 minutes. The enzymes get inactivated by this heating step. After heat inactivation of the enzymes, the solutions were cooled to 4°C. Similarly, the samples of various extents of cross-linking were collected after each LOX addition step and a gap of 30 minutes.

### SDS-PAGE

50 µL of each 20X diluted time point sample was mixed with 50 µL of SDS-PAGE sample buffer (2X Laemmli sample buffer, Bio-Rad) in which 0.1 M of DTT was also added. The above mixture was then heated at 90°C for 10 minutes and cooled down to room temperature with vortex mixing. Next, 20 µL of the above mixture was loaded in the stain free gels (Mini-Protean TGX stain free precast gels, Any kD, Bio-Rad) and the gel was then immersed in the TGS running buffer (25 mM Tris-192 mM Glycine- 0.1 % w/v SDS, pH 8.3). Electrophoresis was done at 300 V for 18 minutes and then the gel was imaged using Gel Doc EZ Imager (Bio-Rad). The relative molar mass of the separated bands was estimated by comparing them to a molar mass standard in the first lane (Precision Plus Protein Standard, Unstained, Bio-Rad). The amount of protein in each sample lane was quantified by image analysis using Image Lab 5.1 (Bio-Rad). The integrated area under the curve of the intensity vs migration distance corresponding to the selected bands was used as the quantity of protein in that group. The bands were grouped into three categories; monomers (18 - 30 kDa), oligomers (30 - 150 kDa) and polymers (> 150 kDa). The polymer fraction was so large that it did not enter the gel and remained in the pockets.

### Absorbance measurement in UV-Vis Spectrophotometer

1 mL of each 20X diluted time point sample from the Na-Caseinate XL series was carefully transferred to a disposable UV-Vis cuvette and gently tapped to remove any air bubbles. The absorbance at 280 nm, 318 nm and 600 nm was measured using a UV-vis spectrophotometer (UV-1800, Shimadzu).

### Fluorescence measurement

A series of dilutions with MQ-water for each 20X diluted time point sample from the Na-Caseinate XL series was prepared in a 96 well plate (black bottom, Thermo scientific). The fluorescence measurements were carried out by using an excitation wavelength of 320 nm and recording the emission spectra at 410 nm, 460 nm, 480 nm, 520 nm and 590 nm in a 96 well plate fluorescence reader (Fluostar Omega, BMG Labtech).

### Film formation and 'cold' gelation of Na-caseinate-lignosulfonate polymers

The casein-lignosulfonate covalently conjugate polymers were tested for their film formation and 'cold' gelation properties. These properties were compared against a blank sample that contained the same concentration of Na-caseinate and Na-lignosulfonate and had gone through the same heating step as the test sample, but no XL reaction was performed on this blank. To 1 mL of each sample, 10 µL of CaCl₂ (50% w/v) was added and the Eppendorf was vortexed for 2 minutes. After resting the samples for 5 minutes, the Eppendorfs were photographed to observe the film formation (wetting) on the inner side of the Eppendorf tube walls above the liquid surface. Next, additional 10 µL of CaCl₂ was added to the same solution followed by vortex mixing for 2 minutes and then kept at rest for 5 minutes. The Eppendorf tubes were inverted after each CaCl₂ addition, to observe if a gel was formed. A photograph was taken when a gel was formed.

### Microencapsulation by W/W phase separation

The cross-linked Na-caseinate-lignosulfonate heteropolymers, also named Na-caseinate-lignosulfonate heteropolymer, were prepared as described above. Na-alginate solution of 30 g/L was prepared in sodium phosphate (NaP) buffer (200 mM, pH 7.0) and once fully dissolved, it was diluted to the required concentration e.g. 20 g/L using the buffer. Next, 0.5 ± 0.05 g of sodium alginate solution was weighed in a 2 mL Eppendorf tube. In another Eppendorf tube (2 mL), 0.5 ± 0.05 g of cross-linked Na-caseinate-lignosulfonate heteropolymer was taken. Then the Na-alginate solution was added to the heteropolymer solution and mixed using a clean plastic spatula for 5 minutes, followed by vortex mixing for 5 minutes. For microscopic observation of the microcapsules formed by W/W phase separation (coacervation), 10 µL of the above solution was placed on a microscope glass slide and covered with a glass cover slip. It was observed using 40X magnification lens and an image was captured. The above procedure was repeated with probiotic bacteria cells such as with *Lactobacillus animalis* cells dispersed in the heteropolymer phase and the microscopic images were saved.

### Matrix encapsulation and cell counting by flow cytometry

The probiotic bacteria cell concentrates such as *Lactobacillus animalis* cell concentrates were formulated with a cryoprotectant (trehalose), antioxidant (sodium ascorbate) and various matrix ingredients (e.g. maltodextrin DE 12 (MD) or a mixture of sodium alginate (Alg) with sodium caseinate (Cas) or sodium lignosulfonate (LS) or Cas + LS or cross-linked heteropolymers) as indicated in **table 2.** All formulation ingredients were heat sterilized before matrix encapsulation. The formulated cells were then deep-frozen in liquid nitrogen in the form of pellets. These pellets were freeze-dried (FD) to obtain a dry powder. The FD powder was sealed in aluminum bags and stored in deep freeze until flow cytometry analysis. For flow cytometry analysis, a weighed amount of FD powder (duplicates) was dispersed in the diluent and injected into flow cytometry for cell counting, following the standard method typically used for lactic acid bacteria. The average total cell counts, and standard deviation was calculated from the duplicates.

**Table 2: Details of formulation used for matrix encapsulation/microencapsulation of the probiotic bacteria cells such as Lactobacillus animalis cells.**

| | Control (% w-DM/% w-DM) | Test (% w-DM/% w-DM) |
|---|---|---|
| Cells | 48.6 | 46.6 |
| Trehalose | 22.3 | 22.3 |
| Ascorbate | 5.8 | 5.8 |
| Maltodextrin | 22.3 | - |
| Caseinate or Na-caseinate-lignosulfonate heteropolymer | - | 12.1-13.4 |
| Alginate | - | 8.9 |
| Sodium lignosulfonate | - | 1.3-0 |
| Salts | Rest | Rest |

### Example 3: results

### Use of LOX-HRP combination for cross-linking milk proteins

Na-caseinate has a very flexible conformation in solution i.e. it behaves as a random coil. The substrate amino acids are expected to be very accessible in the case of Na-caseinate. Caseins present in Na-caseinate were found to be polymerized as the cross-linking reaction progressed **(****figure 2****).** The monomeric bands (18 - 30 kDa) progressively decreased with sequential addition of lactose and at the same time, oligomeric bands (30 - 150 kDa) and polymeric bands (> 150 kDa) appeared. The polymer fraction was so large that it did not enter the gel and remained in the pockets. The polymers survive the reducing (DTT), dissociating (SDS) and heating (90 °C) conditions. These observations prove that the polymerization results from the intermolecular covalent cross-links other than di-sulfide type of cross-links.

It is known in literature that peroxidase can induce di-tyrosine type of cross-links. Therefore, absorbance and fluorescence measurements were performed to identify the type of cross-links being formed during the polymerization of caseins **(****figures 3** and **4****).** It can be seen in **figure 3** that the absorbance at 318 nm increased as compared to the absorbance in the blank sample, while the relative absorbance at 280 and 600 nm did not change. This indicates that the protein concentration as constant in all the samples i.e. concentration was not much changed due to small dilutions after addition of lactose stock solution. The constant absorbance at 600 indicates that the casein polymers being formed most probably have a random coil configuration in solution. If globular polymer shapes were being formed then the absorbance at 600 nm as well as at 280 nm would have increased due to scattering of light. In conclusion, the increase in absorbance at 318 nm is indeed due to the formation of di-tyrosine type of cross-links.

This conclusion was ascertained with the fluorescence measurements **(****figure 4****).** The fluorescence emission spectra after excitation at 320 nm had a peak around 410 nm which is known to be due to di-tyrosine type of cross-links being formed. The biggest fluorescence intensity increase as compared to the blank sample was found to for the sample after first addition of lactose. This relative fluorescence increase of about 15 fold is most probably due to the formation of dimers (di-tyrosine) and most probably oligo-tyrosine type of cross-links are being formed at later stages of cross-linking. The polymerization seems to be of step-growth type, where monomers are converted into dimers, followed by conversion of dimers/monomers into oligomers and eventually cross-linking of oligomers leads to formation of polymers. This can be inferred from the increase and then decrease of the oligomeric fractions in the SDS-PAGE **(****figure 2****).** The fluorescence intensity increased by about 18 times after several lactose additions. The di-tyrosine or oligo-tyrosine cross-links being formed can be expected to be present in many different isomeric forms.

### Use of LOX-HRP combination for cross-linking polyphenols

Na-lignosulfonate was selected as a model polyphenol to test its cross-likability or polymerization after enzymatic treatment with LOX and HRP. The substrate was found to have a very broad range of molecular weights **(****figure 5****).** After enzymatic cross-linking for 5 hours i.e. 10 sequential additions of lactose stock solution, the molecular weights shifted towards higher sizes. This indicates that polyphenols having an accessible phenolic residue in their structure can also be cross-linked through covalent cross-links beings formed (see **figure 1****).** Therefore, a combination of LOX, lactose and a peroxidase can be used to change the polyphenolic profiles of the food products rich in polyphenols.

### Use of LOX-HRP combination for cross-linking proteins with polyphenols

The production of a range of 'new' biopolymers by covalently cross-linking proteins with polyphenols **(****figure 1**) was explored. The first experiment was performed by mixing Na-caseinate and Na-lignosulfonate at a fixed molar ratio **(table 1)** followed by enzymatic modification of this mixture. In this case, the cross-linking reaction was controlled by sequential dosage of LOX **(table 1).** As can be seen in **figure 6****,** the monomeric casein fractions decreased while the oligomeric and polymeric fractions increased with each sequential addition of LOX. For intermediate LOX additions, the oligomeric fraction was found to initially increase and then decrease after longer incubations **(****figure 6****).** These results indicate polymerization of casein. To get an idea of the covalent coupling of lignosulfonate to caseins, the pH of final cross-linked (polymerized) sample was decreased to around 4.5 to precipitate the caseins. As can be seen in the picture **(inset of** **figure 6****),** the brownish color partitions with the precipitated caseins, while the non-attached lignosulfonates remain in the supernatant.

Casein-lignosulfonate polymers should strongly bind calcium (Ca²⁺) ions to the phosphate groups on the caseins and sulfonate groups on the lignosulfonate part of the polymer. These casein-lignosulfonate polymers should also strongly bind to the *in-situ* generated calcium phosphate (CaP) particles by the same mechanism. The interactions between casein-lignosulfonate covalently conjugate polymers and CaP were compared against the blank which contained exactly same concentration of each ingredient, but they were not enzymatically modified. The viscosity was found to significantly increase after *in-situ* generation of CaP particles (visual observation). This increased viscosity led to formation of a uniform film on the Eppendorf tube surface in the case of casein-lignosulfonate heteropolymers **(****figure 7****).** Monomeric casein is known to be surface active and can adsorb at hydrophobic surfaces. But the binding of unmodified casein on the hydrophobic Eppendorf tube surface was not strong enough to sustain a thick film of the composite material. However, the casein-lignosulfonate polymers had much stronger interaction with both the hydrophobic Eppendorf tube surface as well as with CaP particle surface. This led to formation of a thick film on the hydrophobic surface **(****figure 7****).** When the concentration of the *in-situ* generated CaP particles was increased, it led to formation of a shear-thinning gel in the case of casein-lignosulfonate heteropolymers **(****figure 7****).** No gel was formed in the blank sample.

### Use of Na-caseinate-lignosulfonate heteropolymers for microencapsulation of probiotic bacteria

The Na-caseinate-lignosulfonate heteropolymers (2 % w/v) were found to be phase separate (coacervate) from Na-alginate (1 % w/v) to form a W/W emulsion **(****figure 8A****).** The shape of the microcapsules was found to be distorted when probiotic bacteria cells such as *Lactobacillus animalis* cells were mixed with the heteropolymers before coacervation **(****figure 8B****).** The microscopic image shows that most of the cells were encapsulated inside the microcapsules.

The encapsulation efficiency seems to be > 99 % based on the ratio of the cell count in the case of the cross-linked polymers to that of the other types of matrixes. In conclusion, maltodextrin or alginate and caseinate /lignosulfonate-based FD granules are dissolving well in the diluent used for flow cytometry analysis, but, the XL-caseinate-lignosulfonate based microparticles are not fully disintegrated in the diluent.

### REFERENCES

Strauss et al., 2004. Plant phenolics as cross-linkers of gelatin gels and gelatin-based coacervates for use as food ingredients. Food Hydrocolloids 18: 81-89.

### ITEMS

1. A method for encapsulating a bioactive agent such as a microorganism, said method comprising the steps of:
   i) providing the bioactive agent, a heteropolymer obtained by cross-linking of a protein comprising at least one aromatic amino acid with a phenolic compound such as a polyphenolic compound, and a polymer, said polymer having the ability to phase separate from or with said heteropolymer, preferably having the ability to coacervate or to form a complex with said heteropolymer;
   ii) contacting said bioactive agent with said heteropolymer,
   iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent,
   thereby obtaining a product comprising heteropolymer particles encapsulating said bioactive agent.
2. The method according to item 1, further comprising a step of recovering the phase comprising the heteropolymer particles.
3. The method according to any one of the preceding items, wherein the heteropolymer particles are in the dispersed phase.
4. The method according to any one of the preceding items, wherein the polymer is a biopolymer.
5. The method according to any one of the preceding items, further comprising inducing ionic cross-linking of said heteropolymer with a multivalent ion such as a calcium ion before or after step iii).
6. The method according to any one of the preceding items, wherein the bioactive agent is a microorganism such as a bacterium, a probiotic bacterium, a probiotic microorganism, a spore forming bacterium, a lactic acid bacterium or combinations thereof.
7. The method according to any one of the preceding items, wherein the bioactive agent is an ion or an ion salt, optionally wherein said ion or ion salt is used for cross-linking the said heteropolymer.
8. The method according to any one of the preceding items, wherein the heteropolymer is made by a method comprising the steps of:
   a) providing a protein comprising at least one aromatic amino acid, and a phenolic compound;
   b) contacting said protein and said phenolic compound with a carbohydrate substrate such as lactose, a peroxidase (EC 1.11.1.7), and H₂O₂ or an oxidase selected from a lactose oxidase (EC 1.1.99.18) and a hexose oxidase such as a glucose oxidase (EC 1.1.3.4)
   c) incubating said protein and phenolic compound with said carbohydrate substrate, said peroxidase, and said H₂O₂ or oxidase, whereby the oxidase catalyzes conversion of the carbohydrate substrate to a corresponding organic acid and H₂O₂ in the presence of oxygen, whereby the peroxidase catalyzes cross-linking of said phenolic compound using said H₂O₂ as a co-substrate to obtain heteropolymers of said phenolic compound and said protein,
      thereby obtaining heteropolymers.
9. The method according to any one of the preceding items, wherein the heteropolymer formation, the phase separation, and the encapsulation of the bioactive agent are done simultaneously, preferably wherein the phase separation is coacervation.
10. The method according to any one of the preceding items, wherein the protein is comprised within a first substrate.
11. The method according to any one of the preceding items, wherein the phenolic compound is comprised within a second substrate.
12. The method according to any one of the preceding items, wherein the first and the second substrate are one same substrate.
13. The method according to any one of the preceding items, wherein the protein is a milk protein such as a casein, whey protein, or the protein is a plant protein, a fish protein or an animal protein.
14. The method according to any one of the preceding items, wherein the phenolic compound is a plant phenolic compound, such as a phenolic compound from a grain such as a cereal, a bean such as a coffee bean, a leaf such as a tea leaf, a vegetable pulp or a vegetable peel such as from a tuberculous vegetable, or an animal phenolic compound, such as a phenolic compound from an insect, a mammal or a fish, such as a phenolic compound derived from side streams or waste streams from food or feed or paper or wood processing industry, such as a lignin or a lignosulfonate.
15. The method according to any one of the preceding items, wherein the polymer is an alginate, preferably a sodium-alginate, a xyloglucan, a polymerized casein glycomacropeptide, a chitosan, a starch, a modified starch, a food gum, a food stabilizer or a food hydrocolloid.
16. The method according to any one of the preceding items, wherein the heteropolymer is a Na-caseinate-lignosulfonate heteropolymer.
17. The method according to any one of the preceding items, wherein the method further comprises coating the heteropolymer particles with one or more layers of a protective material such as a fat blend and/or an ethylcellulose and/or a hydrophobically modified biopolymer and/or a film forming polymer.
18. The method according to any one of the preceding items, wherein the heteropolymer is obtained by cross-linking comprising the formation of covalent bonds of type C-C, C-O-C, C-N, C-S, S-S, wherein covalent cross-links are formed enzymatically and/or nonenzymatically.
19. The method according to any one of the preceding items, wherein the carbohydrate substrate is comprised within the first and/or the second substrate.
20. The method according to any one of the preceding items, wherein the oxidase and/or the peroxidase are comprised within the first and/or the second substrate.
21. The method according to any one of the preceding items, wherein the peroxidase is lactoperoxidase, horseradish peroxidase, lignin peroxidase, Coprinus peroxidase or myeloperoxidase, preferably lactoperoxidase or horseradish peroxidase.
22. The method according to any one of the preceding items, wherein the oxidase is a cellobiose oxidase.
23. The method according to any one of the preceding items, wherein the carbohydrate substrate is lactose and the acid is lactobionic acid, or wherein the carbohydrate substrate is glucose and the acid is gluconic acid, or wherein the carbohydrate substrate is galactose and the acid is galactonic acid, or wherein the carbohydrate substrate is maltose and the acid is maltobionic acid, or wherein the carbohydrate substrate is xylose and the acid is xylonic acid, or wherein the carbohydrate substrate is cellobiose and the acid is cellobionic acid, or wherein the carbohydrate substrate is mannose and the acid is mannonic acid, or wherein the carbohydrate substrate is fructose and the acid is fructonic acid, preferably the carbohydrate substrate is lactose and the acid is lactobionic acid.
24. The method according to any one of the preceding items, wherein the first substrate is a dairy product.
25. The method according to any one of the preceding items, wherein the substrate is a yogurt, quark, a cheese such as a soft cheese, a drinking yogurt, a cheese spread, skyr or milk, such as soy milk, sheep milk, goat milk, buffalo milk, yak milk, lama milk, camel milk or cow milk, or a combination thereof, optionally supplemented with plant material.
26. The method according to any one of the preceding items, wherein the second substrate comprises in the range of 0.01% to 30% w/w of phenolic compound, such as 0.05%, 1%, 5%, 10%, 15%, 20%, 25% w/w, for example between 2.5 and 6% w/w, such as 3.5% w/w.
27. The method according to any one of the preceding items, wherein the concentration of oxidase is in the range of 0.0001 to 30 U/g, such as 0.01 U/g, 0.05 U/g, or 0.15 U/g, for example between 0.001 and 12.5 U/g, such as between 0.005 and 10 U/g, for example between 0.01 and 7.5 U/g, such as between 0.05 and 5 U/g, for example between 0.1 and 2.5 U/g, such as between 0.15 and 1 U/g, for example between 0.25 and 0.75 U/g, such as 0.5 U/g.
28. The method according to any one of the preceding items, wherein the concentration of peroxidase is in the range of 0.001 to 5000 U/g, such as 5, 15, 30, or 50 U/g, for example between 0.01 and 250 U/g, such as between 0.05 and 125 U/g, for example between 0.1 and 100 U/g, such as between 0.5 and 75 U/g, for example between 1 and 50 U/g, such as between 5 and 40 U/g, for example between 10 and 30 U/g, for example 15, 20 or 25 U/g.
29. The method according to any one of the preceding items, wherein step c) is performed at a temperature of 4°C to 75°C, such as between 4°C and 72°C, for example between 4°C and 70°C, such as between 4°C and 65°C, for example between 4°C and 60°C, such as between 4°C and 55°C, for example between 4°C and 50°C, such as between 4°C and 45°C, for example between 4°C and 40°C, such as between 4°C and 37°C, for example between 4°C and 35°C, such as between 4°C and 30°C, for example between 4°C and 25°C, such as between 4°C and 20°C, for example between 4°C and 15°C, such as between 4°C and 10°C, or such as between 10°C and 75°C, for example between 15°C and 75°C, such as between 20°C and 75°C, for example between 25°C and 75°C, such as between 30°C and 75°C, for example between 35°C and 75°C, such as between 37°C and 75°C, for example between 40°C and 75°C, such as between 45°C and 75°C, for example between 50°C and 75°C, such as between 55°C and 75°C, for example between 60°C and 75°C, such as between 65°C and 75°C, for example between 72°C and 75°C, such as at 75°C, 72°C, 40°C, 37°C, 25°C or 4°C.
30. The method according to any one of the preceding items, wherein step c) is performed for a duration of between 15 seconds and 144 hours, such as between 30 seconds and 132 hours, for example between 1 minute and 120 hours, such as between 2 minutes and 108 hours, for example between 5 minutes and 96 hours, such as between 10 minutes and 84 hours, for example between 20 minutes and 72 hours, such as between 30 minutes and 60 hours, for example between 1 hour and 48 hours, such as between 2 hours and 44 hours, for example between 3 hours and 40 hours, such as between 3 hours and 36 hours, for example between 4 hours and 32 hours, such as between 4 hours and 28 hours, for example between 5 hours and 24 hours, such as between 5 hours and 20 hours, for example between 6 hours and 16 hours, such as between 6 hours and 12 hours, for example between 1 hour and 10 hours, such as between 2 hours and 8 hours, for example between 3 hours and 6 hours, such as 3, 4, 5 or 6 hours.
31. The method according to any one of the preceding items, wherein the pH of the substrate in any of steps a), b) or c) is in the range of 1 to 12, such as between 3.5 to 8.5, such as between 4.0 and 8.0, for example between 4.5 and 7.5, such as between 5.0 and 7.2, for example between 5.5 and 7.0, such as between 6.0 and 6.9, for example between 6.2 and 6.8, such as between 6.4 and 6.7, for example 6.6.
32. The method according to any one of the preceding items, further comprising further cross-linking said heteropolymer and/or said polymer by:
   - contacting said heteropolymer and/or polymer with an enzyme such as a microbial transglutaminase, a polyphenol oxidase, a tyrosinase, a laccase, and/or
   - contacting said polymer with an enzyme such as a microbial transglutaminase, a polyphenol oxidase, a tyrosinase, a laccase, or with a reagent such as genipin, before formation of said heteropolymer, and/or
   - contacting said heteropolymer and/or polymer with a reagent such as genipin or glutaraldehyde.
33. The method according to any one of the preceding items, wherein the process of encapsulation of the bioactive agent is combined with any other process of encapsulation or microencapsulation or entrapment or immobilization such as emulsification, such as sol-gel process, such as gelation, such as microgel formation etc.
34. The method according to any one of the preceding items, further comprising a step of pasteurization or a step of sterilization of any one or all of the bioactive agent, the heteropolymer, the protein, the phenolic compound or the polymer, prior to encapsulation of said bioactive agent.
35. The method according to any one of the preceding items, wherein the averaged degree of polymerization (DP) of the heteropolymer is from 2 to 100000, such as from 3 to 100000, such as from 5 to 1000, such as from 8 to 200, such as from 9 to 150, such as 100 or 125.
36. The method according to any one of the preceding items, wherein the phenolic compound is lignin, lignosulfonate, caffeic acid, cholorogenic acid, a flavonoid, a flavonol, quercetin, rutin, tannic acid, vanillin, p-coumaric acid, ferulic acid or ABTS.
37. The method according to any one of the preceding items, further comprising the step of providing an antioxidant such as ascorbate and/or a cryoprotectant such as a disaccharide, preferably trehalose, in step i) or ii), wherein the antioxidant and/or the cryoprotectant are comprised in the same phase as the heteropolymer particles after step iii).
38. The method according to any one of the preceding items, further comprising the step of processing the product in a food product, a feed product, a pharmaceutical product, a cosmetic product, a seed health product or a plant health product.
39. The method according to any one of the preceding items, wherein the heteropolymer particles have a size between from 10 nm to 10 mm, preferably from 1000 nm to 5000 µm, more preferably from 10 µm to 1000 µm.
40. The method according to any one of the preceding items, wherein at least 0.001% of the bioactive agent is encapsulated, such as at least 0.01%, such as at least 0.1%, such as at least 1%, such as at least 2%, such as at least 3%, such as at least 4%, such as at least 5%, at least 10%, at least 15%, at least 20%, at least 25%, at least 30%, at least 35%, at least 40%, at least 45%, at least 50%, at least 55%, at least 60%, at least 65%, at least 70%, at least 99% or more.
41. A method for making a modified food, feed, plant health, seed health, cosmetic or pharmaceutical product comprising heteropolymer particles encapsulating a bioactive agent such as a microorganism, said method comprising the steps of:
   i) providing a bioactive agent such as a microorganism, a heteropolymer obtained by cross-linking of a protein comprising at least one aromatic amino acid with a phenolic compound such as a polyphenolic compound, and a polymer such as a biopolymer, said polymer having the ability to phase separate from or with said heteropolymer, preferably having the ability to coacervate or to form a complex with said heteropolymer;
   ii) contacting said bioactive agent with said heteropolymer,
   iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent,
      wherein
      - if the protein is provided in a first substrate and the phenolic compound is provided in a second substrate, one of the first and second substrate is the food to be modified, steps ii) and iii) are performed within said first or second substrate, and the modified food, feed, plant health, seed health, cosmetic or pharmaceutical product is obtained in step iii); or
      - if the protein and the phenolic compound are provided in a composition, steps ii) and iii) are performed within said composition, a composition comprising heteropolymers encapsulating said bioactive agent is obtained in step iii), and the method further comprises a step of:
         iv) adding the composition comprising heteropolymer particles encapsulating said bioactive agent to a food, feed, plant health, seed health, cosmetic or pharmaceutical product to be modified;
   thereby obtaining a modified food, feed, plant health, seed health, cosmetic or pharmaceutical product comprising heteropolymer particles encapsulating said bioactive agent.
42. The method according to item 41, wherein steps i), ii) and iii) are performed as defined in any one of items 1 to 40, and wherein said bioactive agent, said microorganism, said heteropolymer, said phenolic compound, said polymer, said phase separation and/or said heteropolymer particles are as defined in any one of items 1 to 40.
43. A composition comprising heteropolymer particles of a phenolic compound and a protein, and a bioactive agent encapsulated therein, obtainable by the method according to any one of the preceding items, preferably wherein the bioactive agent is a microorganism, more preferably wherein the bioactive agent is a microorganism selected from a bacterium, a probiotic bacterium, a probiotic microorganism, a spore forming bacterium, a lactic acid bacterium or combinations thereof.
44. A modified product comprising heteropolymer particles of a phenolic compound and a protein, and a bioactive agent encapsulated therein, obtainable by the method according to any one of the preceding items, wherein the modified product is a modified food, feed, plant health, seed health, cosmetic or pharmaceutical product.
45. The composition according to item 44, wherein said bioactive agent, said microorganism, said heteropolymer, said phenolic compound, said polymer, said phase separation and/or said heteropolymer particles are as defined in any one of items 1 to 40.
46. The modified product according to any one of items 44 to 45, which is a food product or feed product.
47. The modified product according to any one of items 44 to 46, wherein the product is a dairy product, such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material..
48. The modified product according to any one of items 44 to 47, wherein the product is a fermented milk product.
49. The modified product according to any one of items 44 to 48, wherein the product is a pharmaceutical product such as a medicament, a tooth paste or a bone cement.
50. The modified product according to any one of items 44 to 49, wherein the heteropolymer particles have a size between from 10 nm to 10 mm, preferably from 1000 nm to 5000 µm, more preferably from 10 µm to 1000 µm.
51. A method for delivering a bioactive agent to a subject, said method comprising administering to said subject the modified food, feed, cosmetic, plant health, seed health or pharmaceutical product according to any one of items 44 to 50.
52. The method according to item 51, wherein said bioactive agent is as defined in any one of items 1 to 40.
53. A method according to any of items 1 to 42 or 51 to 52, or a composition according to any of items 43 or 45, or a modified product according to item 44 to 50, wherein the bioactive agent is a cell (such as a cell from a microorganism) and a dry product such as a powder is obtained, at least 1.10⁵ cells may be encapsulated per g of dry product, such as at least 1.10⁶, 1.10⁷, 1.10⁸, 1.10⁹, 1.10¹⁰, 1.10¹¹ cells per g of dry product, or more.
54. A method for encapsulating a bioactive agent, said method comprising the steps of:
   i) providing the bioactive agent, a heteropolymer obtained by cross-linking of a protein comprising at least one aromatic amino acid with a phenolic compound such as a polyphenolic compound, and a polymer, said polymer having the ability to phase separate from or with said heteropolymer, preferably having the ability to coacervate or to form a complex with said heteropolymer;
   ii) contacting said bioactive agent with said heteropolymer;
   iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent, thereby obtaining a product comprising heteropolymer particles encapsulating said bioactive agent, wherein the bioactive agent is a microorganism.
55. The method according to item 1, wherein the heteropolymer particles are in the dispersed phase.
56. The method according to any one of the preceding items, wherein the bioactive agent is a microorganism selected from a bacterium, a probiotic bacterium, a probiotic microorganism, a spore forming bacterium, a lactic acid bacterium or combinations thereof.
57. The method according to any one of the preceding items, wherein the heteropolymer is made by a method comprising the steps of:
   a) providing a protein comprising at least one aromatic amino acid, and a phenolic compound;
   b) contacting said protein and said phenolic compound with a carbohydrate substrate such as lactose, a peroxidase (EC 1.11.1.7), and H₂O₂ or a cellobiose oxidase (EC 1.1.99.18) or
   c) incubating said protein and phenolic compound with said carbohydrate substrate, said peroxidase, and said H₂O₂ or cellobiose oxidase, whereby the cellobiose oxidase catalyzes conversion of the carbohydrate substrate to a corresponding organic acid and H₂O₂ in the presence of oxygen, whereby the peroxidase catalyzes cross-linking of said phenolic compound using said H₂O₂ as a co-substrate to obtain heteropolymers of said phenolic compound and said protein, thereby obtaining heteropolymers.
58. The method according to any one of the preceding items, wherein the heteropolymer formation, the phase separation, and the encapsulation of the bioactive agent are done simultaneously, preferably wherein the phase separation is coacervation, and wherein the bioactive agent is a microorganism.
59. The method according to any one of the preceding items, wherein the protein is a milk protein such as a casein, whey protein, or the protein is a plant protein, a fish protein or an animal protein, and/or wherein the phenolic compound is a plant phenolic compound, such as a phenolic compound from a grain such as a cereal, a bean such as a coffee bean, a leaf such as a tea leaf, a vegetable pulp or a vegetable peel such as from a tuberculous vegetable, or an animal phenolic compound, such as a phenolic compound from an insect, a mammal or a fish, such as a phenolic compound derived from side streams or waste streams from food or feed or paper or wood processing industry, such as a lignin or a lignosulfonate.
60. The method according to any one of the preceding items, wherein the polymer is an alginate such as a sodium-alginate, a xyloglucan, a polymerized casein glycomacropeptide, a chitosan, a starch, a modified starch, a food gum, a food stabilizer or a food hydrocolloid, and/or
   wherein the heteropolymer is a Na-caseinate-lignosulfonate heteropolymer, and/or preferably wherein the polymer is an alginate and the heteropolymer is a Na-caseinate-lignosulfonate heteropolymer, and/or more preferably wherein the polymer is sodium-alginate and the heteropolymer is a Na-caseinate-lignosulfonate heteropolymer.
61. The method according to any one of the preceding items, wherein the method further comprises coating the heteropolymer particles with one or more layers of a protective material such as a fat blend and/or an ethylcellulose and/or a hydrophobically modified biopolymer and/or a film forming polymer.
62. The method according to any one of the preceding items, wherein the peroxidase is lactoperoxidase, horseradish peroxidase, lignin peroxidase, Coprinus peroxidase or myeloperoxidase, preferably lactoperoxidase or horseradish peroxidase (HRP).
63. The method according to any one of the preceding items, further comprising a step of pasteurization or a step of sterilization of any one or all of the bioactive agent, the heteropolymer, the protein, the phenolic compound or the polymer, prior to encapsulation of said bioactive agent, wherein the bioactive agent is a microorganism.
64. The method according to any one of the preceding items, wherein the averaged degree of polymerization (DP) of the heteropolymer is from 2 to 100000, such as from 3 to 100000, such as from 5 to 1000, such as from 8 to 200, such as from 9 to 150, such as 100 or 125.
65. The method according to any one of the preceding items, further comprising the step of providing an antioxidant such as ascorbate and/or a cryoprotectant such as a disaccharide, preferably trehalose, in step i) or ii), wherein the antioxidant and/or the cryoprotectant are comprised in the same phase as the heteropolymer particles after step iii).
66. The method according to any one of the preceding items, further comprising the step of processing the product in a food product, a feed product, a pharmaceutical product, a cosmetic product, a seed health product or a plant health product, preferably further comprising the step of processing the product in a fermented milk product or in a dairy product such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material.
67. A composition, such as a food composition or a feed composition, comprising heteropolymer particles of a phenolic compound and a protein, and a bioactive agent encapsulated therein, obtainable by the method according to any one of the preceding items, preferably wherein the bioactive agent is a microorganism, more preferably wherein the bioactive agent is a microorganism selected from a bacterium, a probiotic bacterium, a probiotic microorganism, a spore forming bacterium, a lactic acid bacterium or combinations thereof.
68. The composition according to the preceding claim, wherein the composition is a fermented milk product or a dairy product, such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material.

## Claims

1. A method for encapsulating a bioactive agent, said method comprising the steps of:
i) providing the bioactive agent, a heteropolymer obtained by cross-linking of a protein comprising at least one aromatic amino acid with a phenolic compound such as a polyphenolic compound, and a polymer, said polymer having the ability to phase separate from or with said heteropolymer, preferably having the ability to coacervate or to form a complex with said heteropolymer;
ii) contacting said bioactive agent with said heteropolymer;
iii) inducing phase separation, such as coacervation or complex coacervation, of the heteropolymer from or with the polymer, to obtain a continuous phase and a dispersed phase, wherein one of continuous phase and of the dispersed phase comprises heteropolymer particles encapsulating said bioactive agent, thereby obtaining a product comprising heteropolymer particles encapsulating said bioactive agent, wherein the bioactive agent is not a microorganism.

2. The method according to claim 1 wherein the bioactive agent is selected from the group consisting of phytochemicals, vitamins, minerals, peptides, bacteriocins, enzymes, nucleic acids and ion salts.

3. The method according to claim 2, wherein the bioactive agent is an enzyme, such as lysozyme, lactase or a dairy enzyme such as chymosin.

4. The method according to any one of the preceding claims, wherein the heteropolymer particles are in the dispersed phase.

5. The method according to any one of the preceding claims, wherein the bioactive agent is a microorganism selected from a bacterium, a probiotic bacterium, a probiotic microorganism, a spore forming bacterium, a lactic acid bacterium or combinations thereof.

6. The method according to any one of the preceding claims, wherein the heteropolymer is made by a method comprising the steps of:
a) providing a protein comprising at least one aromatic amino acid, and a phenolic compound;
b) contacting said protein and said phenolic compound with a carbohydrate substrate such as lactose, a peroxidase (EC 1.11.1.7), and H₂O₂ or a cellobiose oxidase (EC 1.1.99.18) or
c) incubating said protein and phenolic compound with said carbohydrate substrate, said peroxidase, and said H₂O₂ or cellobiose oxidase, whereby the cellobiose oxidase catalyzes conversion of the carbohydrate substrate to a corresponding organic acid and H₂O₂ in the presence of oxygen, whereby the peroxidase catalyzes cross-linking of said phenolic compound using said H₂O₂ as a co-substrate to obtain heteropolymers of said phenolic compound and said protein, thereby obtaining heteropolymers.

7. The method according to any one of the preceding claims, wherein the heteropolymer formation, the phase separation, and the encapsulation of the bioactive agent are done simultaneously, preferably wherein the phase separation is coacervation.

8. The method according to any one of the preceding claims, wherein the protein is a milk protein such as a casein, whey protein, or the protein is a plant protein, a fish protein or an animal protein, and/or wherein the phenolic compound is a plant phenolic compound, such as a phenolic compound from a grain such as a cereal, a bean such as a coffee bean, a leaf such as a tea leaf, a vegetable pulp or a vegetable peel such as from a tuberculous vegetable, or an animal phenolic compound, such as a phenolic compound from an insect, a mammal or a fish, such as a phenolic compound derived from side streams or waste streams from food or feed or paper or wood processing industry, such as a lignin or a lignosulfonate.

9. The method according to any one of the preceding claims, wherein the polymer is an alginate such as a sodium-alginate, a xyloglucan, a polymerized casein glycomacropeptide, a chitosan, a starch, a modified starch, a food gum, a food stabilizer or a food hydrocolloid, and/or
wherein the heteropolymer is a Na-caseinate-lignosulfonate heteropolymer, and/or preferably wherein the polymer is an alginate and the heteropolymer is a Na-caseinate-lignosulfonate heteropolymer, and/or more preferably wherein the polymer is sodium-alginate and the heteropolymer is a Na-caseinate-lignosulfonate heteropolymer.

10. The method according to any one of the preceding claims, wherein the method further comprises coating the heteropolymer particles with one or more layers of a protective material such as a fat blend and/or an ethylcellulose and/or a hydrophobically modified biopolymer and/or a film forming polymer.

11. The method according to any one of the preceding claims, wherein the peroxidase is lactoperoxidase, horseradish peroxidase, lignin peroxidase, Coprinus peroxidase or myeloperoxidase, preferably lactoperoxidase or horseradish peroxidase (HRP).

12. The method according to any one of the preceding claims, further comprising a step of pasteurization or a step of sterilization of any one or all of the bioactive agent, the heteropolymer, the protein, the phenolic compound or the polymer, prior to encapsulation of said bioactive agent.

13. The method according to any one of the preceding claims, wherein the averaged degree of polymerization (DP) of the heteropolymer is from 2 to 100000, such as from 3 to 100000, such as from 5 to 1000, such as from 8 to 200, such as from 9 to 150, such as 100 or 125.

14. The method according to any one of the preceding claims, further comprising the step of providing an antioxidant such as ascorbate and/or a cryoprotectant such as a disaccharide, preferably trehalose, in step i) or ii), wherein the antioxidant and/or the cryoprotectant are comprised in the same phase as the heteropolymer particles after step iii).

15. The method according to any one of the preceding claims, further comprising the step of processing the product in a food product, a feed product, a pharmaceutical product, a cosmetic product, a seed health product or a plant health product, preferably further comprising the step of processing the product in a fermented milk product or in a dairy product such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material.

16. A composition, such as a food composition or a feed composition, comprising heteropolymer particles of a phenolic compound and a protein, and a bioactive agent encapsulated therein, obtainable by the method according to any one of the preceding claims.

17. The composition according to the preceding claim, wherein the composition is a fermented milk product or a dairy product, such as a yogurt, quark, a cheese such as a soft cheese, a drinking yogurt, a cheese spread, skyr or milk, preferably the milk is soy milk, sheep milk, goat milk, buffalo milk, yak milk, lama milk, camel milk or cow milk or a combination thereof, optionally supplemented with plant material.
